# FASCICULE DE BREVET EUROPEEN

(11) **EP 1 907 861 B1**
(45) Date de publication et mention de la délivrance du brevet: **24.03.2010**
(21) Numéro de dépôt: 06778816.6
(22) Date de dépôt: 07.07.2006
(51) Int. Cl.: G01N 33/68

(54) **PROCÉDÉ DE DÉTECTION DES FPCA UTILISANT UN AGENT D'AGRÉGATION DES FPCA ET UN AGENT DE CAPTURE DES AGRÉGATS FORMÉS**
VERFAHREN ZUM NACHWEIS AGGREGATBILDENDER ZIRKULIERENDER PROTEINFORMEN UND MITTEL ZUM EINFANGEN GEBILDETER AGGREGATE
METHOD FOR DETECTING AGGREGATE-FORMING CIRCULATING PROTEIN FORMS AND AGENT FOR CAPTURING FORMED AGGREGATES

(30) Priorité: 21.07.2005 FR 0507757
(43) Date de publication de la demande: 09.04.2008
(73) Titulaire: Centre National de la Recherche Scientifique, 75794 Paris Cedex 16 (FR); bioMérieux S.A., 69280 Marcy l'Etoile (FR); UNIVERSITE CLAUDE BERNARD - LYON 1, 69622 Villeurbanne Cédex (FR)
(72) Inventeur: EVENO-NOBILE, Anne, F38850 Bilieu (FR); COLEMAN, Anthony, William, F-69300 Caluire (FR); CECILLON, Sébastien, F-38110 Dolomieu (FR); PERRON, Hervé, F-69290 Saint Genis les Ollières (FR); RODRIGUE, Marc, F-69290 Grezieu La Varenne (FR)
(74) Mandataire: Bitaud, Valérie Marie-Odile
(86) Numéro de dépôt international: PCT/FR2006/001641
(87) Numéro de publication internationale: WO 2007/010110

(56) Documents cités:
- WO-A-03/073106
- WO-A-2004/059321
- WO-A-2004/059322
- FR-A- 2 865 280
- US-B1- 6 365 414
- ANONYMOUS: "Amyloid"[Online] 17 mars 2003 (2003-03-17), XP002375713 Extrait de l'Internet: URL:http://web.archive.org/web/20050319085 527/http://en.wikipedia.org/wiki/Amyloid> [extrait le 2006-04-03]
- PAUDEL H K ET AL: "Heparin-induced conformational change in microtubule-associated protein Tau as detected by chemical cross-linking and phosphopeptide mapping." THE JOURNAL OF BIOLOGICAL CHEMISTRY. 19 MAR 1999, vol. 274, no. 12, 19 mars 1999 (1999-03-19), pages 8029-8038, XP002374773 ISSN: 0021-9258
- MEMMI L ET AL: "Protein-calixarene interactions: complexation of Bovine Serum Albumin by sulfonatocalix[n]arenes." CHEMICAL COMMUNICATIONS (CAMBRIDGE, ENGLAND) 7 DEC 2001, no. 23, 7 décembre 2001 (2001-12-07), pages 2474-2475, XP002374774 ISSN: 1359-7345
- Philipp Fresenius: "Organic chemical nomenclature" Elis Horwood Limited , page 195, 226
- INOUE ET AL.: "Structural and spectroscopic studies of a metacyclophane and its binuclear Cu2+ complex" INORGANICA CHIMICA ACTA, vol. 317, 2001, pages 320-323,

## Description

La présente invention concerne le domaine des maladies neurodégénératives reconnues non infectieuses à ce jour, et en particulier un procédé de détection des formes protéiques circulantes d'agrégation liées à ces maladies.

L'accroissement des maladies liées au vieillissement touche de plus en plus nos populations dont la moyenne d'âge a augmenté fortement durant le dernier siècle. Certaines de ces maladies touchent le cerveau et semblent affecter toutes les fonctions cognitives. Des atteintes des fonctions sensitivo-motrices peuvent aussi être importantes dans certaines de ces maladies. Ce sont les maladies dites neurodégénératives, reconnues non infectieuses à ce jour, telles que la maladie de Parkinson, la maladie d'Alzheimer, les tauopathies, les démences à corps de Lewy, la maladie de Huntington et les démences vasculaires.

Ainsi, par exemple, plus de 25 millions de personnes dans le monde souffrent de la maladie d'Alzheimer. En France, près de 800 000 personnes sont atteintes de la maladie d'Alzheimer ou de maladies apparentées. En outre, le vieillissement démographique de la population s'accentue et va conduire à une augmentation de ce nombre. On compte déjà près de 165 000 nouveaux malades par an. La maladie d'Alzheimer représente la quatrième cause de mortalité, après les affections cardiovasculaires, les cancers et les accidents vasculaires cérébraux (AVC). Cette démence neurodégénérative conduit progressivement et irréversiblement à la perte de la mémoire (amnésie) et des fonctions cognitives (aphasie, apraxie, agnosie).

Comme la maladie d'Alzheimer, les principales maladies neurodégénératives non infectieuses sont caractérisées par des dépôts extracellulaires et/ou intracellulaires constitués de protéines non infectieuses qui peuvent être présentes sous forme circulante (formes protéiques circulantes d'agrégation ou FPCA) et sont impliquées dans des processus d'agrégation pathologique du système nerveux central. Ces protéines peuvent subir des modifications conformationelles spontanées, induites par des co-facteurs ou des enzymes, ou encore par des mutations génétiques qui favorisent leur agrégation spontanée sous forme de dépôts protéiques ; ces modifications entraînent une accumulation dans différentes zones cérébrales selon le type de protéine et selon les circonstances physiopathologiques (El-Agnaf O et al., 2003, Lancet Neurol, 2 : 461-462).

Ainsi, dans le cas de la maladie d'Alzheimer, les protéines agrégées sont principalement la protéine Tau et des fragments de la protéine APP (Amyloid Precursor Protein), les peptides bêta-Amyloïdes (bêta 1-40, bêta 1-41, bêta 1-42, par exemple). La protéine Tau est également associée aux tauopathies, démences non-Alzheimer associées à des dépôts de protéine Tau agrégée dans le cerveau en l'absence de protéine bêta-Amyloïde. La protéine APP et/ou ses fragments peptidiques bêta-Amyloïde sont également associés aux démences vasculaires, caractérisées par des dépôts péri-vasculaires d'agrégats amyloïde. Dans le cas de la maladie de Parkinson, caractérisée par une dégénérescence neuronale au niveau des noyaux gris centraux, une protéine associée est la Parkine, notamment pour les formes familiales, ainsi que l'alpha-Synucléine. Cette dernière est aussi impliquée dans la démence Parkinsonnienne qui peut survenir en surcroît des troubles sensitivo-moteurs. L'apha-Synueléine est aussi associée aux démences à corps de Lewy. Ces dernières sont caractérisées par un dépôt de protéine alpha-Synucléine agrégée, notamment dans des zones du cortex frontal, ce qui engendre une dégénérescence neuronale avec atteinte des fonctions cognitives débouchant sur un syndrome démentiel. Dans le cas de la maladie de Huntington, la protéine agrégeante est la Huntingtine. Ainsi, ce phénomène de dépôts cérébraux d'agrégats protéiques neurotoxiques est retrouvé dans la plupart des maladies neurodégénératives mais, contrairement aux maladies dites « à prions », les protéines associées aux maladies neurodégénératives ne présentent pas de propriétés infectieuses et ne sont donc pas transmissibles. En effet, les protéines « Prion » sont les agents vecteurs des encéphalopathies spongiformes transmissibles et, par injection de ces prions pathogènes isolés chez des individus malades, on peut transmettre la maladie ; ceci est aussi valable pour les maladies à prions d'origine génétique, comme la maladie de Gertsmann-Straüssler-Scheinker (GSS), dont le prion pathologique est initié par les mutations génétiques de l'hôte mais dont la protéine pathologique, une fois formée, acquiert les propriétés infectieuses propres aux maladies à prions (Lantos PL., 1992, Histopathology, 20(1) : 1-11). Les «maladies à prions » résultent de l'accroissement progressif de la quantité de protéine prion pathogène chez l'individu inoculé ou contaminé avec ces dernières, ou encore porteur des mutations génétiques capables d'initier la conversion pathologique de la protéine normale, créant ainsi une « infection endogène ».

Les protéines associées aux maladies neurodégénératives non infectieuses se différencient notamment de ces dernières par leur incapacité à transmettre la maladie par injection systémique à un individu sain. De même, aucun élément tissulaire issu de ces maladies neurodégénératives non infectieuses n'a pu transmettre la maladie, ce qui exclut que ces dernières soient des maladies infectieuses, contrairement aux maladies à prions.

Le diagnostic actuel de ces maladies neurodégénératives non infectieuses repose le plus souvent sur l'imagerie, qui complète l'examen clinique et neuropsychologique. C'est une procédure lourde et onéreuse qui requiert plusieurs étapes de diagnostic, aucune n'étant effectuée sur un échantillon biologique. Dans quelques cas, une mutation d'origine génétique est à l'origine de l'agrégation anormale d'une de ces protéines, un diagnostic génétique au niveau des séquences de l'ADN est possible, mais cela reste peu fréquent au regard des formes « non-génétiques ».

Un diagnostic de ces maladies plus aisé et plus rapide est donc nécessaire. Le diagnostic dans des échantillons biologiques chez l'homme devient donc d'une extrême importance. Notamment, leur détection immunologique permettrait un diagnostic plus simple, voire plus précoce, avec un gain évident sur les conduites thérapeutiques à initier avant l'extension des lésions (El-Agnaf O et al., 2003, *supra*).

Plusieurs équipes se sont attachées à une telle détection dans des échantillons biologiques. Ainsi, la demande de brevet EP 713 095 décrit le diagnostic dans le liquide céphalorachidien (LCR) d'un patient atteint de la maladie d'Alzheimer par mesure de la quantité d'un peptide de β-amyloïde, éventuellement en association avec la mesure de la quantité de la protéine Tau, et comparaison à une valeur prédéterminée. Cette méthode a pour inconvénient de n'être démontrée que pour le LCR et que la détection de peptide de β-Amyloïde doit y être complétée par un dosage de protéine Tau, pour améliorer la spécificité du test (Sjogren M et al., 2003, Clin Chim Acta, 332:1-10).

Il n'existe pas à l'heure actuelle de diagnostic biologique de routine sanguin pour ces maladies neurodégénératives non infectieuses. Par exemple, la protéine Tau est difficile à détecter dans le sang, puisqu'elle n'est produite que dans les neurones et se retrouve très diluée dans le sang, après passage dans le LCR. D'ailleurs, dans ce dernier, la protéine Tau est plus ou moins bien détectée par les tests existants. La mise en oeuvre du dosage de la protéine APP et de ses fragments circulants, dont la protéine précurseur, peut être produite dans les plaquettes sanguines, reste aussi problématique car de nombreuses FPCA sont liées à des protéines plasmatiques qui peuvent masquer des épitopes et, ainsi, les rendre indétectables et/ou non capturables par des anticorps dirigés contre ces mêmes épitopes. Il n'est donc pas possible, puisque les épitopes masqués sont ceux que reconnaissent les anticorps utilisés, d'utiliser des procédures immunoenzymatiques classiques de type « ELISA sandwich ». De plus, les anticorps sont de très grosses protéines qui ne peuvent accéder à des sites moléculaires des FPCA, si ceux-ci sont internalisés dans un réseau tri-dimensionnel comme c'est le cas dans les formes agrégées et dans les formes associées à des ligands plasmatiques. Seules de petites molécules peuvent interagir avec un nombre accru de protéines dans ces FPCA agrégées, alors que des anticorps détecteront plus facilement les formes libres non agrégées et non associées à des ligands plasmatiques, c'est-à-dire la fraction la plus physiologique de ces protéines associées aux maladies neurodégénératives. Ainsi, une fraction variable et dont la significativité reste invérifiable de ce fait, ne peut pas être dosée par les tests immunologiques standards (Kuo Y et al., 2000, Biochem Biophys Res Commun, 268:750-756).

C'est pourquoi les méthodes de l'état de la technique se heurtent sans arrêt à la difficulté de capturer, de concentrer et/ou de détecter les formes solubles et/ou circulantes des protéines associées aux pathologies neurodégénératives non infectieuses. Des méthodes adaptées à chaque type de protéine sont parfois envisagées mais, outre le fait qu'elles sont difficilement transposables en test de routine à grande échelle, elles ne permettent pas de mettre en oeuvre une méthode unique et commune à toutes ces protéines, ou à un nombre suffisant de protéines qui serait acceptable en diagnostic (Kuo Y et al., 2000, *supra*).

Une méthode capable de recueillir ces protéines, de les séparer des fluides biologiques sans passer par une reconnaissance anticorps, puis éventuellement de les traiter pour dissocier les ligands éventuels dans des conditions compatibles avec leur immobilisation sur des surfaces adaptées aux tests immunoenzymatiques et, après, de les identifier enfin avec des anticorps, permettrait de mettre en oeuvre une détection efficace et comparable de ces différentes protéines. De telles conditions seraient à meme de permettre un isolement plus efficace de la totalité de ces protéines et de leurs différentes formes circulantes, avec une interprétation homogène des dosages qui corrèle mieux les paramètres biocliniques réels, donc les diagnostics respectifs de ces maladies.

Le brevet US 6 365 414 décrit un procédé *in vitro* permettant la détection de la formation de peptides beta-amyloïde, et utilisant des cations de métaux lourds tels que le zinc. Cependant, en raison du faible nombre de sites de fixation du zinc sur les protéines, cette technique ne permet pas d'agréger et de concentrer les peptides beta-amyloïde en formant des réseaux multimoléculaires sédimentables par centrifugation à basse vitesse.

La demande de brevet WO 03/073106 décrit un procédé de liaison sélective des formes pathologiques des protéines prion, amyloïde et Tau. En particulier, la capture de la forme agrégée de la protéine Tau par le dextran sulfate est décrite dans l'un des exemples de cette demande. Ce document ne mentionne pas l'utilisation d'un agent d'agrégation et le dextran sulphate n'appartient ni à la famille des glycosaminoglycanes ni à celle des molécules macrocycliques.

La demande de brevet WO2004059321 décrit un procédé de détection de la protéine prion utilisant un aminoglycoside pour précipiter la protéine.

Les présents inventeurs ont maintenant mis en évidence contre toute attente que l'utilisation d'un agent I non protéique produisant l'agrégation des formes circulantes des protéines non infectieuses impliquées dans des processus d'agrégation pathologique du système nerveux central choisi parmi la rifampicine, l'isoniazide, l'éthambutol, la triéthylènetétraamine (TET), la bis-3-aminopropylamine, le tétrachlorhydrate de spermine, le sesquisulfate de dihydrostreptomycine et la streptomycine et/ou d'un agent II non protéique de capture des agrégats naturels de FPCA ou induits par lesdits agents I qui est un métacyclophane ou un glycosaminoglycane, dans un test pour le diagnostic des formes protéiques circulantes d'agrégation (FPCA) liées aux maladies neurodégénératives non infectieuses, permettait la détection, selon une seule méthode, de ces formes protéiques, à des dilutions et dans des conditions où elles ne sont pas détectables avec les méthodes utilisées actuellement. L'utilisation de ces deux agents seuls ou en combinaison peut améliorer nettement, mais en tout cas n'empêche pas, la capacité de liaison des formes protéiques d'agrégation à un partenaire de liaison spécifique de ces formes protéiques d'agrégation utilisé dans le test diagnostic.

La présente invention a pour objet un procédé de détection d'au moins une FPCA liée aux maladies neurodegénératives non infectieuses dans un échantillon biologique d'origine humaine susceptible de contenir desdites FPCA, lesdites FCPA contenant:
a) des acides aminés dont les chaînes latérales ont soit une fonction acide, soit une fonction accepteur de liaison hydrogène, et qui sont présents globalement au moins 4 fois dans une séquence peptidique de longueur inférieure ou égale à 20 acides aminés et
b) un minimum de trois acides aminés basiques dans une séquence peptidique de longueur inférieure ou égale à quinze acides aminés,
**caractérisé en ce qu'**il met en oeuvre un agent I non protéique produisant l'agrégation des formes circulantes des protéines non infectieuses impliquées dans des processus d'agrégation pathologique du système nerveux central, qui est choisi parmi la rifampicine, l'isoniazide, l'éthambutol, la triéthylènetétramine, la bis-3-aminopropylamine, le tétrachlorhydrate de spermine, le sesquisulfate de dihydrostreptomycine et la streptomycine, et un agent II non protéique de capture des agrégats naturels de FPCA ou induits par lesdits agents I, qui est un métacyclophane ou un glycosaminoglycane, et en ce qu'il met en oeuvre la révélation de la présence desdits FCPA.

La présente invention a aussi pour objet un procédé de détection d'au moins une FPCA liées aux maladies neurodegénératives non infectieuses, lesdites FCPA contenant des acides aminés dont les chaînes latérales ont soit une fonction acide, soit une fonction accepteur de liaison hydrogène, et qui sont présents globalement au moins 4 fois dans une séquence peptidique de longueur inférieure ou égale à 20 acides aminés,
**caractérisé en ce qu'**il comprend l'étape de mise en présence d'un échantillon biologique, issu ou obtenu d'un organisme humain, avec un agent I non protéique tel que défini ci-dessus et en ce qu'il met en oeuvre la révélation de la présence desdits FCPA.

Elle concerne également l'utilisation d'un kit de diagnostic comprenant un agent I non protéique produisant l'agrégation des formes circulantes des protéines non infectieuses impliquées dans des processus d'agrégation pathologique du système nerveux central et éventuellement un agent II non protéique de capture des agrégats naturels de FPCA ou induits par lesdits agents I pour la détection d'au moins une FPCA liée aux maladies neurodégénératives non infectieuses lesdits agents I et II étant tels que décrits précédemment.

Le procédé de l'invention est donc un procédé simple, universel en terme d'application, et particulièrement utile pour effectuer un diagnostic dans des échantillons biologiques, tels que le sang, des maladies neurodégénératives non infectieuses, quelle que soit la nature des FPCA et quelle que soit leur concentration.

Selon un premier mode de réalisation, il met en oeuvre deux agents, à savoir :
- un agent I non protéique produisant l'agrégation des formes circulantes des protéines non infectieuses impliquées dans des processus d'agrégation pathologique du système nerveux central et
- un agent II non, protéique de capture des agrégats naturels de FPCA ou induits par lesdits agents I lesdits agents I et II étant tels que décrits précédemment.

Par agent non protéique produisant l'agrégation des formes circulantes des protéines non infectieuses impliquées dans des processus d'agrégation pathologique du système nerveux central, on entend toute molécule de nature non protéique capable d'entraîner en sa présence l'agrégation des FPCA, c'est-à-dire capable de produire des amas protéiques de taille supérieure aux FPCA initiaux et/ou de permettre leur sédimentation rapide par simple centrifugation. L'agrégation est en effet définie comme étant la formation de réseaux multi-moléculaires sédimentables par centrifugation à basse vitesse (10000g).

Par agent non protéique de capture des agrégats naturels de FPCA ou induits par lesdits agents I, on entend toute molécule de nature non protéique capable de se lier aux agrégats de FPCA, qu'ils soient formés de façon naturelle, c'est-à-dire dans l'organisme, ou artificielle, c'est-à-dire après réaction avec les agents I.

Par maladie neurodégénérative reconnue non infectieuse à ce jour, on entend une maladie caractérisée par des dépôts extracellulaires et/ou intracellulaires de FPCA, comme décrit précédemment, ces FPCA n'étant pas des protéines infectieuses, c'est-à-dire des protéines transmissibles. Ainsi, les maladies à prion, telle que la maladies de Creutzfeldt-Jakob ou l'encéphalopathie spongiforme bovine, sont clairement exclues de cette définition.

A titre d'exemples de maladies incluses dans la définition de l'invention, on peut citer la maladie de Parkinson, la maladie d'Alzheimer, les tauopathies, les démences à corps de Lewy, la maladie de Huntington et les démences vasculaires.

Les échantillons biologiques dans lesquels le procédé de détection de l'invention est mis en oeuvre sont tout échantillon d'origine humaine susceptible de contenir au moins une FPCA.

A titre d'exemple de tels échantillons, on peut citer le cerveau, les tissus du système nerveux central, les organes tels que la rate et l'intestin, ainsi que les fluides biologiques tels que le liquide céphalo-rachidien, l'urine et le sang, ces derniers étant préférés et le sang constituant un échantillon biologique de choix.

Du fait de la caractéristique particulière des agents I à permettre l'agrégation des FPCA et à les capturer de façon amplifiée dans l'échantillon à tester par rapport aux agents protéiques tels que les anticorps (capture de plus de formes), il est possible d'utiliser uniquement l'agent I pour détecter les FPCA.

Ainsi, l'invention concerne également un procédé de détection des FPCA liées aux maladies neurodégénératives non infectieuses tel que défini dans la revendication 8.

Les agents II non protéiques de capture des agrégats naturels de FPCA ou induits par lesdits agents I sont toutes molécules non protéiques ayant cette fonction de capture choisis parmi les métacyclophanes, qui sont des molécules macrocycliques, et les glycosaminoglycanes.

Les glycosaminoglycanes sont largement connus de l'homme du métier et sont décrits par exemple dans Polysaccharides, M. Yalpani, Elsevier, Amsterdam, 1988.

A titre de glycosaminoglycanes appropriés aux fins de l'invention, on peut citer par exemple l'héparine, le sulfate de chondroïtine, le sulfate de dermatane, l'acide hyaluronique et le sulfate de kératane.

Par molécule macrocyclique, on entend un composé constitué d'une succession de cycles formant un macrocycle.

Les molécules macrocycliques ont pour avantage particulier qu'elles permettent de piéger la protéine à tester, sous forme libre ou sous forme d'agrégat, par un effet cage.

Les molécules macrocycliques peuvent être préparées selon les techniques connues de l'homme du métier, par exemple décrites dans Comprehensive Supramolecular Chemistry, Pergamon, Oxford, 1996.

Les molécules macrocycliques du procédé de l'invention sont choisies parmi les métacyclophanes, les calix-arènes étant particulièrement préférés. De tels composés calix-arènes peuvent être obtenus suivant la méthodologie décrite dans Arduini, A. et al., 1996, Macrocycle Synthesis, Eds. Harwood, L.M. & Moddy,C.J. Oxford University Press, Oxford et Da Silva et al., 2001, J. Supramol. Chem., 1 :135-138.

Selon un mode de réalisation préféré, le métacyclophane est le p-sulphonato-3,7-(2-aminoéthyloxy)-calix-[6]-arène.

Selon un mode de réalisation, la molécule macrocyclique répond à la formule générale (I) suivante : 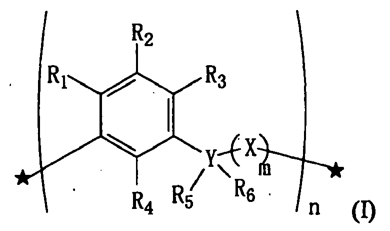 dans laquelle
R₁ représente un atome d'hydrogène, un groupe hydroxyle, un groupe OR ou un groupe OCOR, R étant tel que défini ci-dessous,
R₂ représente un atome d'hydrogène, un groupe R, COR, Pol, CH₂Pol, dans lesquels Pol représente un groupe phosphate, sulfate, amine, ammonium, acide carboxylique et R est tel que défini ci-dessous,
R₃ représente un atome d'hydrogène, un groupe hydroxyle, un groupe OR ou un groupe OCOR dans lesquels R est tel que défini ci-dessous,
R₄ représente un atome d'hydrogène, un groupe hydroxyle, un groupe OR, un groupe OCH₂R ou un groupe OCOR, dans lesquels R est tel que défini ci-dessous,
Y est un atome de carbone, d'azote, ou un atome de soufre,
R₅ et R₆, chacun indépendamment, sont absents ou représentent un atome d'hydrogène, un groupement CH₂ ou R tel que défini ci-dessous, ou bien
R₅ et R₆ représentent ensemble un atome d'oxygène ou de soufre,
X représente un groupement CH₂, ou un atome d'oxygène ou de soufre,
m représente un entier égal à 0 ou 1,
R représente un atome d'hydrogène ou une chaîne hydrocarbonée, saturée ou non, ramifiée ou non, cyclique ou non cyclique, substituée ou non par un groupement halogène, et portant des fonctions polaires ou non polaires,
n est un entier compris entre 3 et 15,
les substituants R₁ à R₅, R, X, Y et l'entier m peuvent être de nature différente suivant les motifs.

Ainsi, le composé de formule (I) se présente sous forme d'une succession de n motifs caractérisés par la présence d'un cycle benzénique, et les substituants de ce cycle peuvent être variables d'un motif à l'autre, dans la limite de leurs définitions ci-dessus.

Bien entendu, la présence des étoiles dans les formules permet de représenter la connexion nécessaire permettant la formation d'un cycle.

Les chaînes hydrocarbonées, saturées ou non, ramifiées ou non, cycliques ou non cycliques, substituées ou non par un groupement halogène, et portant des fonctions polaires ou non polaires, sont largement connues de l'homme du métier. A titre d'exemples, on peut citer les alkyles, les alcènes, les aryles et les cycles saturés tels que le cyclohexane. Un exemple de groupement non polaire est CF₃ et des exemples de groupements polaires sont les substituants Pol tels que définis précédemment.

Les composés de formule (I) particulièrement préférés répondent à la formule (Ia) suivante : 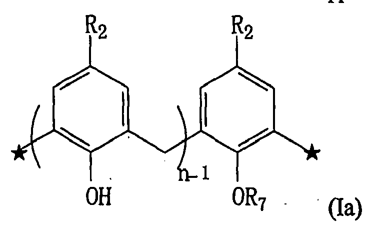 dans laquelle
n est un entier compris entre 4 et 8,
chaque groupement R₂, pris indépendamment, est un groupement sulfate ou un groupement phosphate
R₇ représente un groupement (CH₂)ₜ-(CO)ₛ-(NH₂) ou un groupement (CH₂)ₜ-COOH où t est un entier compris entre 0 et 6 et s est un entier compris entre 0 et 6.

Les composés de formule (Ia) particulièrement préférés sont ceux pour lesquels les deux groupements R₂ sont chacun un groupement sulfate, n est 4, 6 ou 8, et R₇ est un atome d'hydrogène, un groupement -CH₂COOH, un groupement -CH₂CONH₂ ou un groupement -CH₂CH₂NH₂.

Selon un mode de réalisation préféré, le ligand macrocyclique répond à la formule générale (Ia) dans laquelle n=6, R₂=sulfate et R₇ est -CH₂CH₂NH₂.

Du fait de la caractéristique particulière des agents II à permettre la capture des agrégats de FPCA et ce de façon amplifiée dans l'échantillon à tester par rapport aux agents protéiques tels que les anticorps (capture de plus de formes), il est possible d'utiliser uniquement l'agent II pour détecter les FPCA.

Ainsi, la description concerne également un procédé de détection des FPCA liées aux maladies neurodégénératives non infectieuses, **caractérisé en ce qu'**il comprend ou consiste en l'étape de mise en présence d'un échantillon biologique, issu ou obtenu d'un organisme humain, avec un agent II non protéique de capture des agrégats naturels de FPCA, de préférence une molécule macrocyclique ou un glycosaminoglycane.

Bien entendu, les agents II sont tels que définis précédemment.

La quantité d'agents I et II peut être facilement déterminée par l'homme du métier en fonction des spécificités de l'échantillon. Ainsi, par exemple, la quantité de l'agent I, tel que la streptomycine, peut être comprise entre 50 et 500 mg/ml, de préférence entre 100 et 300 mg/ml.

Contre toute attente, les Demanderesses ont mis en évidence que les FPCA à détecter dans le procédé de l'invention, pour se lier aux agents I et en particulier aux agents ayant au moins deux fonctions guanidinium et/ou pyridinium et/ou ammonium, contiennent des acides aminés dont les chaînes latérales ont soit une fonction acide, de préférence acide carboxylique, comme l'acide aspartique (D) ou l'acide glutamique (E), soit une fonction accepteur de liaison hydrogène, comme la sérine (S) ou l'asparagine (N), ces acides aminés (D, E, S ou N) étant présents globalement au moins 4 fois dans une séquence peptidique de longueur inférieure ou égale à 20 acides aminés. De préférence, une densité de ces acides aminés (D, E, S ou N) supérieure ou égale à quatre sur une séquence peptidique inférieure ou égale à 20 acides aminés est retrouvée au moins deux fois sur la séquence complète de la protéine constituant les FPCA. De préférence, ces acides aminés (D, E, S ou N) sont présents globalement au moins 5 fois dans une séquence peptidique de longueur inférieure ou égale à 20 acides aminés ou au moins 4 fois dans une séquence peptidique de longueur inférieure ou égale à 16 acides aminés. De préférence, une densité telle que définie précédemment est associée à une zone de plus forte densité pour ces acides aminés (D, E, S ou N), correspondant à au moins 5 fois dans une séquence peptidique de longueur inférieure ou égale à 12 acides aminés. Une densité de fonctions équivalentes sur une distance spatiale équivalente peut aussi constituer une structure mimétique de celle qui a été définie précédemment dans une structure primaire de protéine.

De même, les Demanderesses ont mis en évidence que, pour se lier aux agents II et en particulier aux molécules telles que les calixarènes, les FPCA contiennent un minimum de trois acides aminés basiques, de préférence l'arginine (R), la lysine (K), l'histidine (H) ou la glutamine (Q), dans une séquence peptidique de longueur inférieure ou égale à douze, voire quinze, acides aminés. Une densité de trois charges positives équivalentes, sur une distance spatiale équivalente, peut aussi constituer une structure mimétique de celle qui a été définie précédemment dans une structure primaire de protéine. Ce peut-être, par exemple, une conformation qui projette ces cations ou leurs équivalents fonctionnels dans un espace tridimensionnel équivalent à une séquence de douze, voire quinze, acides aminés sur une portion de cet espace représentant une hélice alpha constituée d'acides aminés

L'ajout des deux agents I et/ou II à l'échantillon biologique dans ce procédé peut être mis en oeuvre dans n'importe quel ordre, puisque, contre toute attente, cela ne gêne en rien la détection des FPCA, par exemple à l'aide d'un anticorps de détection anti-FPCA. Toutefois, on préfère que ledit agent I soit ajouté audit échantillon biologique pour l'agrégation des FPCA avant ledit agent II, ce qui constitue un mode de réalisation de l'invention.

Selon un mode de réalisation de l'invention, le procédé comprend ou consiste en les étapes consistant à :
a) ajouter audit échantillon ledit agent I pour agréger les FPCA et les précipiter,
b) mettre le mélange ainsi obtenu en présence dudit agent II pour capturer lesdits agrégats de FPCA et
c) révéler la présence des FPCA.

De préférence, pour favoriser la précipitation des FPCA, après ajout de l'agent I, on procède à un chauffage modéré du milieu réactionnel à une température comprise entre 25 et 45°C, une température de 37°C étant préférée.

Les agrégats de FPCA, formés en présence de l'agent I, peuvent être séparés du milieu réactionnel avant leur réaction avec l'agent II. Le procédé de séparation peut être mis en oeuvre par tout procédé de séparation d'un précipité connu de l'homme du métier. A titre d'exemples, les agrégats de FPCA sont séparés du milieu réactionnel par centrifugation ou séparation sur membrane, puis par élimination du surnageant. Cette étape de séparation permet d'éliminer tous produits non nécessaires à la réaction de détection ultérieure des FPCA, tels que l'agent I libre en solution.

Pour favoriser, après formation des agrégats par un agent I, la séparation entre les ligands plasmatiques et les protéines des FPCA sur lesquelles lesdits ligands plasmatiques sont liés (dénaturation), on peut ajouter un agent chimique de dénaturation tel que la Guanidine-HCl, à la concentration de 1 à 6 moles/l et/ou on effectue une dénaturation thermique à 100°C, ce qui qui permet de libérer les épitopes masqués des protéines des FPCA en les séparant des ligands qui ne sont pas liés par l'agent I. Ceci permet d'améliorer davantage la sensibilité du procédé de détection de l'invention. La dénaturation desdits agrégats présents dans l'échantillon biologique à tester avant la réaction des agrégats de FPCA avec l'agent II peut également être mise en oeuvre par tout procédé de dénaturation d'agrégats protéiques connu de l'homme du métier.

Ainsi, le procédé de détection des FPCA selon l'invention comprend de préférence au moins l'une des étapes supplémentaires i) et ii) suivantes consistant à :
i) séparer les agrégats de FPCA du mélange réactionnel et
ii) dénaturer les agrégats de FPCA,
ces étapes étant incluses le cas échéant entre l'étape a) et l'étape b).

La révélation de la présence des FPCA dans un échantillon biologique selon le procédé de l'invention peut être mise en oeuvre selon les procédés classiques de détection d' analytes dans un échantillon.

Elle peut par exemple être mise en oeuvre par une détection immunologique ou non.

Par détection immunologique, on entend la mise en évidence d'une réaction immunologique avec les FPCA, cette réaction immunologique consistant en la liaison entre les FPCA à détecter et au moins un partenaire de liaison spécifique de FPCA ou bien en une réaction de compétition entre les FPCA susceptibles d'être contenues dans l'échantillon à tester et des FPCA marquées.

A titre de détection non immunologique, on peut citer par exemple les techniques de coloration sur gel d'électrophorèse bien connues de l'homme du métier.

La détection des FPCA par réaction immunologique peut-être mise en oeuvre par exemple après addition d'au moins un partenaire de liaison spécifique de FPCA.

Par partenaire de liaison spécifique des FPCA, on entend tout partenaire capable de se lier aux FPCA concernées. La visualisation de la réaction immunologique consistera alors en la visualisation du complexe partenaire de liaison spécifique des FPCA/FPCA.

Selon un mode de réalisation préféré, le procédé de l'invention est tel qu'on ajoute au moins un partenaire de liaison spécifique de FPCA pour la réaction immunologique entre le partenaire de liaison spécifique des FPCA et les FPCA, le cas échéant dans l'étape c). Bien entendu, on peut également ajouter de tels partenaires lorsqu'on décide de n'utiliser que l'agent I dans le procédé de l'invention.

Le nombre de partenaires de liaison différents à ajouter dans le procédé de l'invention dépend du nombre de FPCA différentes à tester. Ainsi, dans le cas de la détection de la maladie d'Alzheimer, si on ne veut tester que la protéine Tau, on ajoutera un partenaire de liaison spécifique de Tau. En revanche, si on veut à la fois détecter la protéine Tau et des peptides bêta-Amyloïde, on ajoutera également des partenaires de liaison spécifiques des peptides bêta-Amyloïde.

A titre de partenaire de liaison spécifique des FPCA, on peut citer, par exemple, les anticorps, les fragments d'anticorps, les polypeptides, les protéines, les acides nucléiques, les haptènes et les aptamères.

Le terme « anticorps » inclut les anticorps polyclonaux ou monoclonaux, les anticorps obtenus par recombinaisons génétiques et des fragments d'anticorps.

Les anticorps polyclonaux peuvent être obtenus par immunisation d'un animal avec au moins un antigène cible d'intérêt, dans le cas présent une FPCA, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment la FPCA concernée.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un antigène cible d'intérêt, le cas présent une FPCA, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

A titre d'anticorps appropriés pour l'invention, on peut citer par exemple les anticorps monoclonaux dirigés contre les protéines Tau totale (anticorps Tau-1 (Chemicon), T46 (Zymed)), contre la protéine Tau phosphorylée (anticorps PHF-6 (Zymed)), contre la protéine APP (anticorps 22C11 (Chemicon)), contre les peptides bêta-Amyloïde (anticorps 6E10 et 4G8 (Sigma)).

Les fragments d'anticorps sont tels qu'ils conservent la fonction de liaison aux FPCA.

Par « polypeptide », on entend un enchaînement d'au moins deux acides aminés. Par acides aminés, on entend les acides aminés primaires qui codent pour les protéines, les acides aminés dérivés après action enzymatique comme la *trans*-4-hydroxyproline et les acides aminés naturels, mais non présents dans les protéines comme la norvaline, la N-méthyl-L-leucine, la staline (Hunt S. dans Chemistry and Biochemistry of the amino acids, Barett GC, ed., Chapman and Hall, London, 1985), les acides aminés protégés par des fonctions chimiques utilisables en synthèse sur support solide ou en phase liquide et les acides aminés non naturels.

Le terme « protéine » inclut les holoprotéines et les hétéroprotéines comme les nucléoprotéines, les lipoprotéines, les phosphoprotéines, les métalloprotéines et les glycoprotéines aussi bien fibreuses que globulaires.

Par acide nucléique, on entend les oligonucléotides, les acides désoxyribonucléiques et les acides ribonucléiques, ainsi que leurs dérivés.

Le terme « oligonucléotide » désigne un enchaînement d'au moins 2 nucléotides (désoxyribonucléotides ou ribonucléotides, ou les deux), naturels ou modifiés. Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine. Pour illustrer une liaison internucléotidique modifiée, on peut mentionner les liaisons phosphorothioate, N-alkylphosphoramidate, alkylphosphonate et alkylphosphodiester. Les alpha-oligonucléotides tels que ceux décrits dans FR-A-2 607 507, les LNA tels que phosphorothioate-LNA and 2'-thio-LNA décrits dans Bioorganic & Medicinal Chemistry Letters, Volume 8, Issue 16, 18 August 1998 ,pages 2219-2222, et les PNA qui font l'objet de l'article de M. Egholm et al., J. Am. Chem. Soc. (1992), 114, 1895-1897, sont des exemples d'oligonucléotides constitués de nucléotides dont le squelette est modifié.

Le terme « haptène » désigne des composés non immunogènes, c'est-à-dire incapables par eux-mêmes de promouvoir une réaction immunitaire par production d'anticorps, mais capables d'êtres reconnus par des anticorps obtenus par immunisation d'animaux dans des conditions connues, en particulier par immunisation avec un conjugué haptène-protéine. Ces composés ont généralement une masse moléculaire inférieure à 3000 Da, et le plus souvent inférieure à 2000 Da, et peuvent être par exemple des peptides glycosylés, des métabolites, des vitamines, des hormones, des prostaglandines, des toxines ou divers médicaments, les nucléosides et les nucléotides.

Les aptamères sont des partenaires de capture de nature protéique et nucléique qui ont pour fonction d'agir en tant qu'anticorps et de se lier à des ligands protéiques (Toulmé, J.J. et Giege, R. , 1998, Medecine Science, 14(2), 155-166).

Ces polypeptides, protéines, haptènes et aptamères ont tous la capacité de se lier aux FPCA ou à l'agrégat de FPCA.

La visualisation de la réaction immunologique entre le ou les partenaires de liaison spécifiques des FPCA et les FPCA mises en oeuvre, notamment dans l'étape c), peut être effectuée par tout moyen de détection connu de l'homme du métier, tels que des moyens directs ou indirects.

Dans le cas de la détection directe, c'est-à-dire sans l'intermédiaire d'un marquage, on observe la réaction immunologique par exemple par résonance plasmon ou par voltamétrie cyclique sur une électrode portant un polymère conducteur.

Dans le cas de la détection indirecte, c'est-à-dire par l'intermédiaire d'un marquage, le marquage peut être effectué par l'intermédiaire dudit partenaire de liaison spécifique des FPCA qui sera alors préalablement marqué.

La visualisation de la présence des FPCA dans un échantillon biologique selon le procédé de l'invention peut également être mise en oeuvre selon une méthode dite de compétition. On ajoute alors, notamment dans l'étape c), à la place du partenaire spécifique de liaison de FPCA, de la FPCA préalablement marquée. Dans ce cas, le signal de détection est maximal en l'absence de FPCA, puis diminue progressivement à mesure que la concentration en FPCA recherchée, non marquée, augmente par la réaction de compétition.

Par marquage, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés, luminescents, colorants,
- les molécules radioactives comme le 32P, le ³⁵S ou le ¹²⁵I,
- les molécules fluorescentes telles que la fluorescéine, la rhodamine, l'alexa ou les phycocyanines, et
- les particules telles que les particules en or, en latex magnétique, les liposomes.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter à l'article J. Histochem. Cytochem. 45 : 481-491, 1997.

Le marquage de protéines est largement connu de l'homme du métier et est décrit par exemple par Greg T. Hermanson dans Bioconjugate Techniques, 1996, Academic Press Inc, 525B Street, San Diego, CA92101 USA.

Selon le type de marquage utilisé, comme par exemple en utilisant une enzyme, l'homme du métier ajoutera des réactifs permettant la visualisation du marquage.

De tels réactifs sont largement connus de l'homme du métier et sont décrits notamment dans Principles and Practice of Immunoessay, 2nd Edition, Edited by C. Price, D.J. Newman Stockton Press, 1997, 345 Park Avenue South, New York.

La détection de FPCA peut être une détection en phase solide, c'est-à-dire utilisant une phase solide sur laquelle est immobilisé un partenaire de capture destiné à la capture de la protéine à détecter. Dans le cas d'un procédé de la présente invention, c'est l'agent II qui peut servir de partenaire de capture préalablement immobilisé sur un support solide. Un exemple de détection en phase solide bien connu de l'homme du métier est la détection de type sandwich, telle que la détection de type ELISA.

Ainsi, selon un mode de réalisation préféré de l'invention, ledit agent II est lié à un support solide.

A titre de support solide, on peut citer par exemple les billes, telles que les billes magnétiques, et les plaques de microtitrage.

L'agent II peut être lié au support solide de façon connue de l'homme du métier, telle que par adsorption ou liaison covalente, la liaison covalente étant préférée.

Ainsi, le support solide peut être fonctionnalisé par une fonction susceptible de former une liaison avec une fonction portée par ledit agent II. Suivant un mode de réalisation préféré, le support solide est fonctionnalisé par une liaison NHS (N-hydroxysuccinimide) ou par une fonction NH₂. Cette fonction peut réagir avec une fonction portée sur l'agent II. Dans ce mode de réalisation, les agents II portant une fonction susceptible de réagir pour former une liaison avec la liaison fonctionnelle du support solide, notamment portant une liaison NH₂ ou COOH, sont particulièrement préférés.

Pour la mise en oeuvre du procédé de détection d'au moins une FPCA de l'invention, on peut utiliser des kits de diagnostic comprenant un agent I et un agent II, lesdits agents étant tels que définis précédemment.

Ainsi, l'invention concerne également l'utilisation d'un kit de diagnostic comprenant un agent I non protéique produisant l'agrégation des formes circulantes des protéines non infectieuses impliquées dans des processus d'agrégation pathologique du système nerveux central et éventuellement un agent II non protéique de capture des agrégats naturels de FPCA pour la détection d'au moins une FPCA liées aux maladies neurodégénératives non infectieuses.

Selon un mode de réalisation préféré, ledit agent II présent dans le kit est lié à un support solide pour la mise en oeuvre de la détection d'au moins une FPCA selon un procédé de détection en phase solide.

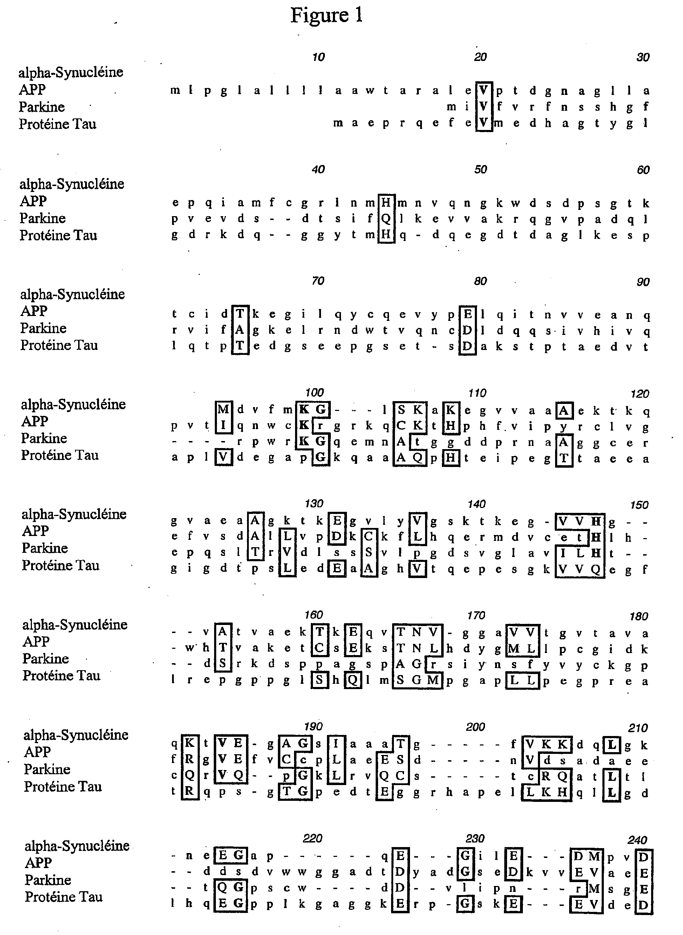

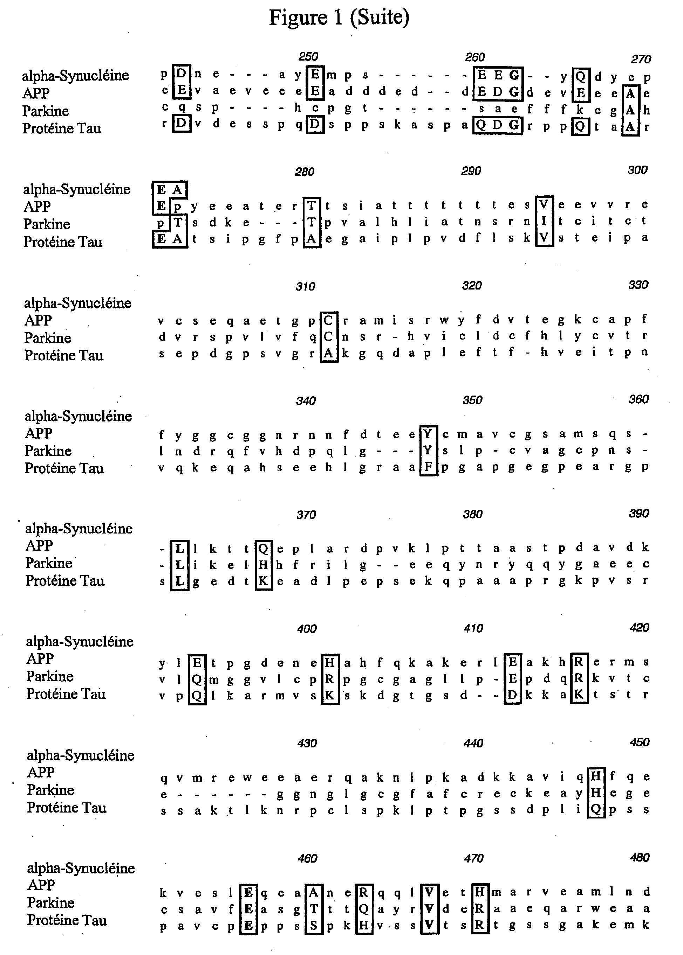

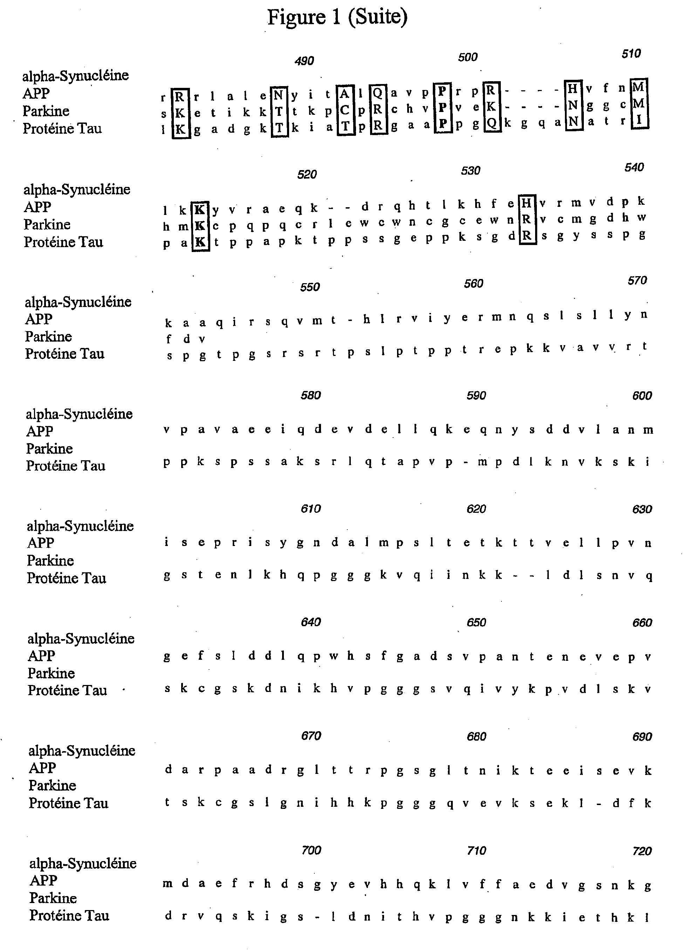

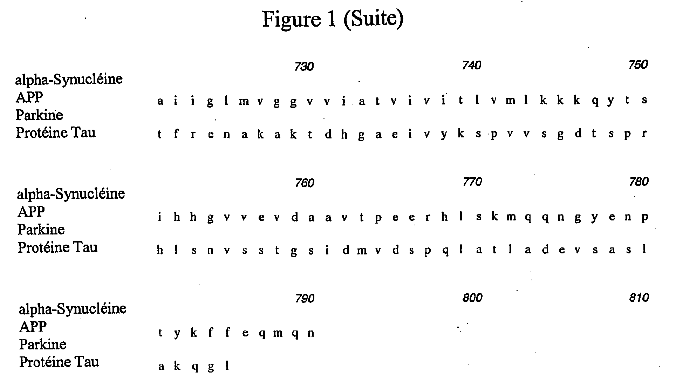

L'invention sera mieux comprise à l'aide des exemples suivants donnés à titre illustratif et non limitatif, ainsi qu'à l'aide des figures 1 à 24, dans lesquelles :
- la figure 1 montre une comparaison des séquences des FPCA alpha-Synucléine, APP (protéine précurseur des peptides Amyloïde), Parkine et protéine Tau ;

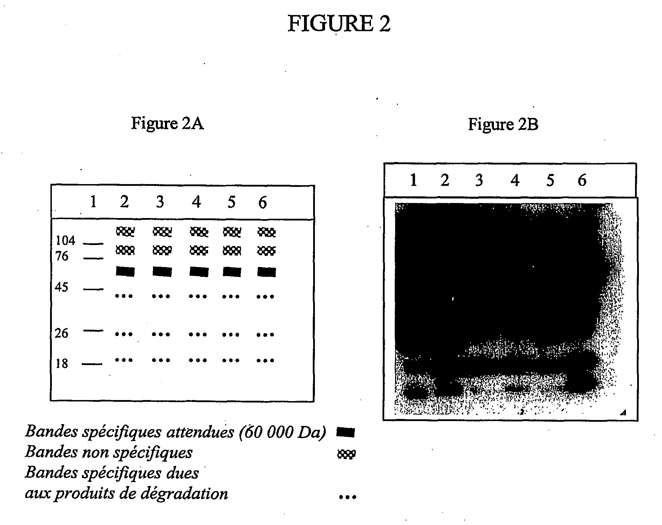

- la figure 2 est une représentation schématique (figure 2A) de la radiographie d'un gel d'électrophorèse (figure 2B) à la suite d'un Western Blot, obtenu après migration de la protéine Tau traitée par un agent I qui est soit la streptomycine (pistes 3 et 4), soit la TET (pistes 5 et 6), la piste 1 correspondant à la piste des marqueurs de poids moléculaire et la piste 2 correspondant à l'échantillon témoin sans traitement par l'agent I,

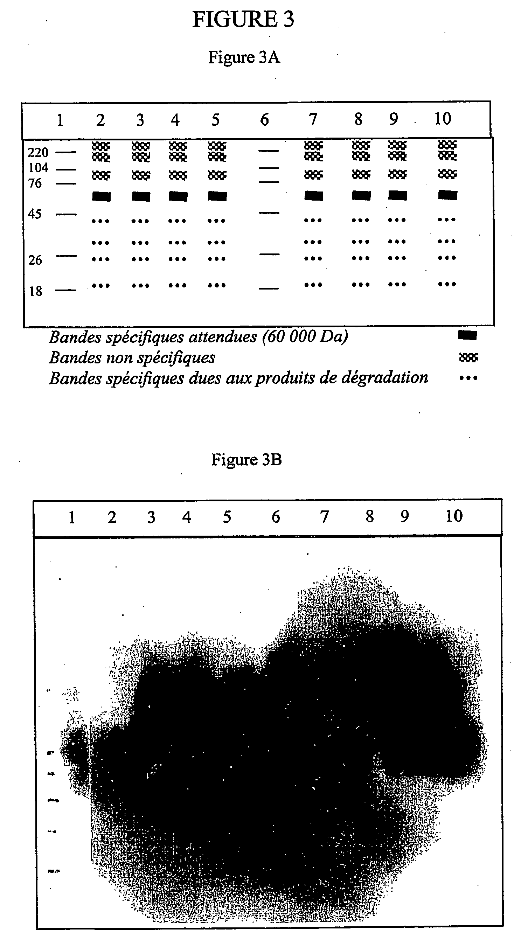

- la figure 3 est une représentation schématique (figure 3A) de la radiographie d'un gel d'électrophorèse (figure 3B) à la suite d'un Western Blot, obtenu après migration de la protéine Tau traitée par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I,

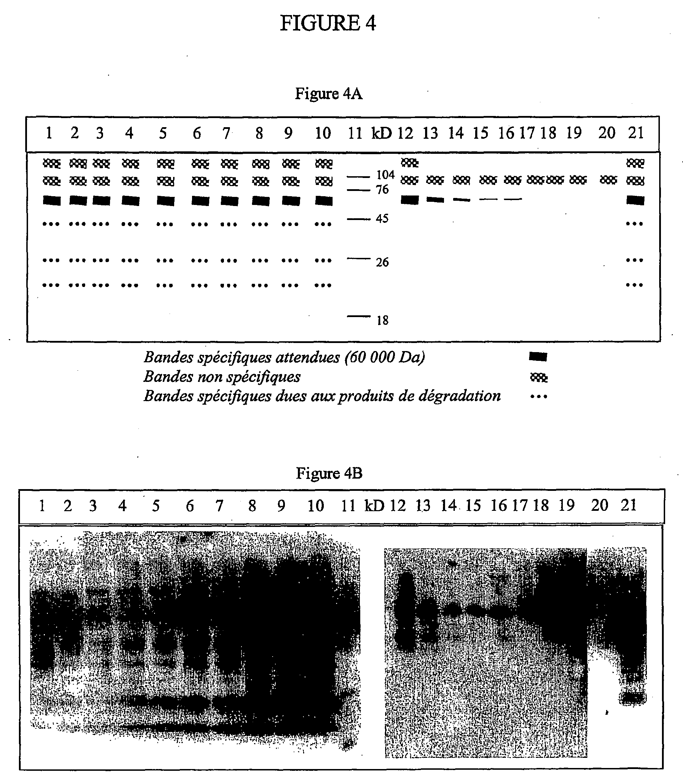

- la figure 4 est une représentation schématique (figure 4A) de la radiographie d'un gel d'électrophorèse (figure 4B) à la suite d'un Western Blot, obtenu après migration de la protéine Tau traitée par le calix-arène p-sulfonato-3,7-(2-aminoéthyloxy)-calix-[6]-arène (C6S) comme agent II, la fraction récupérée étant soit le culot (pistes 2 à 9), soit le surnageant (pistes 12 à 20), les pistes 1 et 21 correspondant à l'échantillon témoin sans traitement par le calix-arène et la piste 11 correspondant à la piste des marqueurs de poids moléculaire,

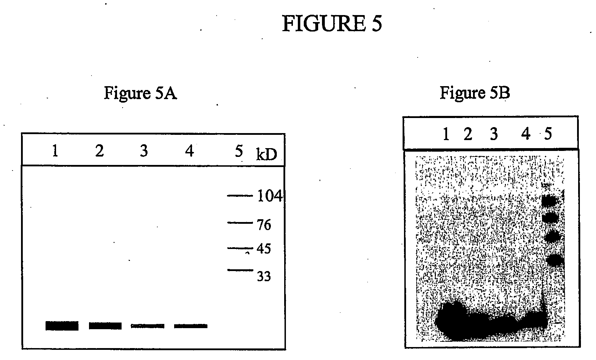

- la figure 5 est une représentation schématique (figure 5A) de la radiographie d'un gel d'électrophorèse (figure 5B) à la suite d'un Western Blot, obtenu après migration du peptide peptide bêta-Amyloïde 1-40 traité par le C6S (piste 2), par la streptomycine (piste 3) ou par la TET (piste 4), la piste 1 correspondant à l'échantillon témoin sans traitement par l'agent I ou II et la piste 5 correspondant à la piste des marqueurs de poids moléculaire,

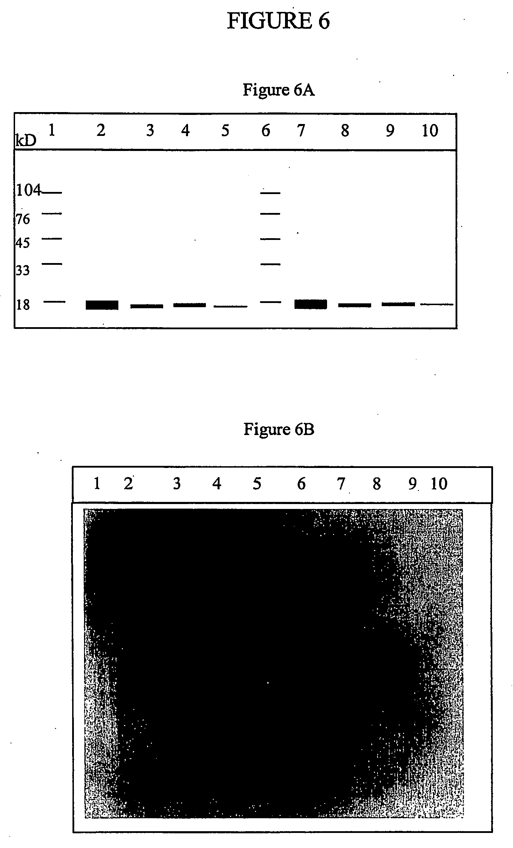

- la figure 6 est une représentation schématique (figure 6A) de la radiographie d'un gel d'électrophorèse (figure 6B) à la suite d'un Western Blot, obtenu après migration du peptide bêta-Amyloïde 1-40 traité par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I,

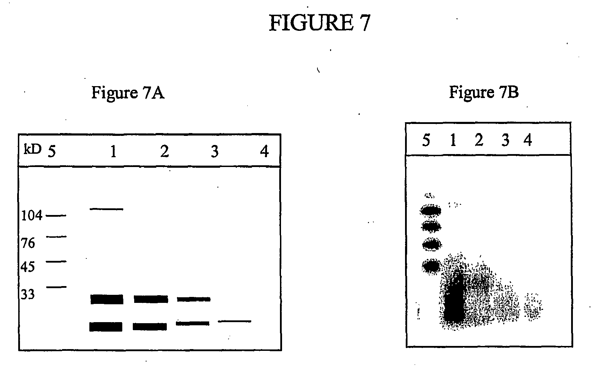

- la figure 7 est une représentation schématique (figure 7A) de la radiographie d'un gel d'électrophorèse (figure 7B) à la suite d'un Western Blot, obtenu après migration du peptide peptide bêta-A 1-42 traité par le C6S (piste 2), par la streptomycine (piste 3) ou par la TET (piste 4), la piste 1 correspondant à l'échantillon témoin sans traitement par l'agent I ou II et la piste 5 correspondant à la piste des marqueurs de poids moléculaire,

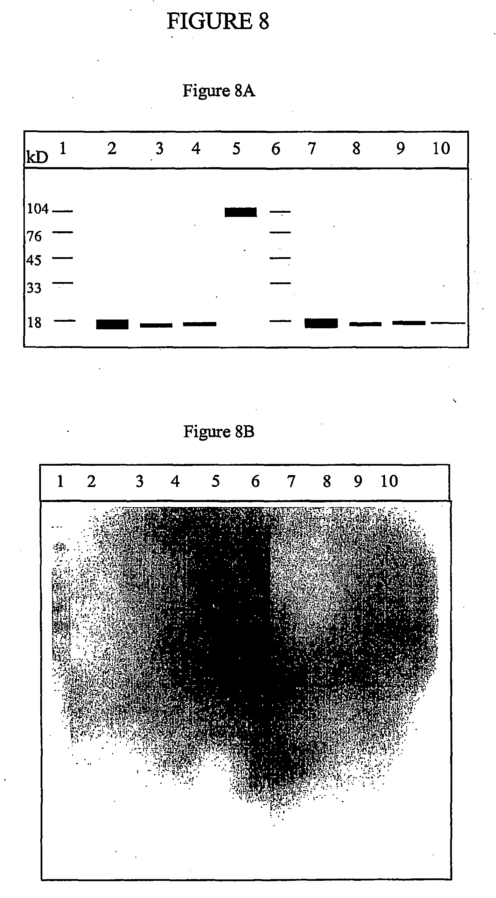

- la figure 8 est une représentation schématique (figure 8A) de la radiographie d'un gel d'électrophorèse (figure 8B) à la suite d'un Western Blot, obtenu après migration du peptide bêta-Amyloïde 1-42 traité par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I,

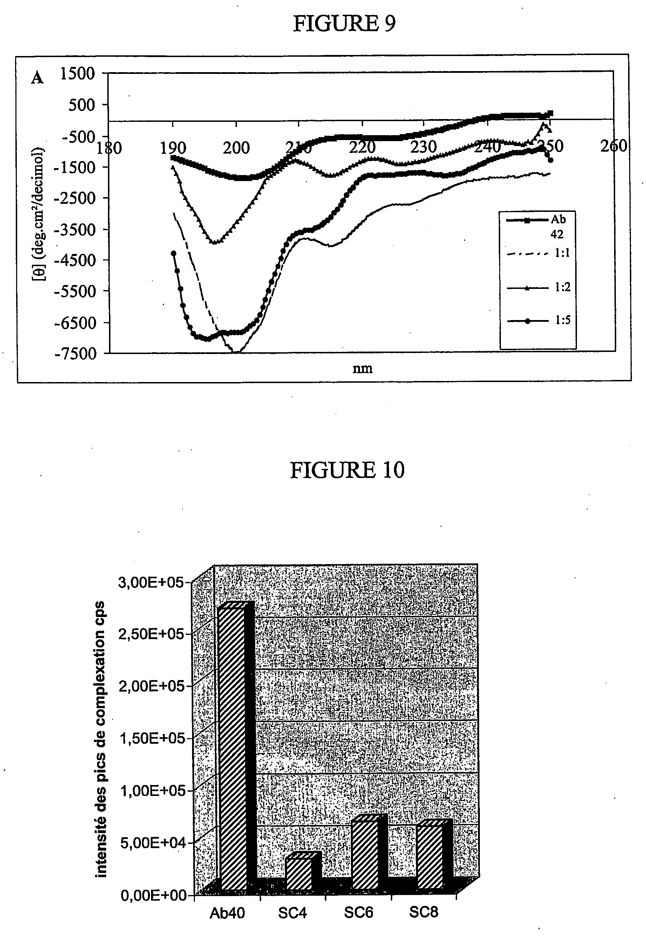

- la figure 9 est une représentation graphique donnant l'élipticité molaire en fonction de la longueur d'onde obtenues par dichroïsme circulaire du peptide bêta-Amyloïde 1-42 après traitement par l'agent II para-sulfonato-calix[4]arène selon différentes concentrations, Ab42 étant le témoin sans traitement par l'agent II,
- la figure 10 est une représentation graphique donnant l'intensité des pics de complexation cps (coup par seconde) obtenue par spectrométrie de masse en mode électrospray du peptide bêta Amyloïde 1-40 après traitement par les agents II : para-sulfonato-calix[4]arène (SC4), para-sulfonato-calix[6]arène (SC6), para-sulfonato-calix[8]arène (SC8), Ab40 étant le témoin sans traitement par l'agent II,

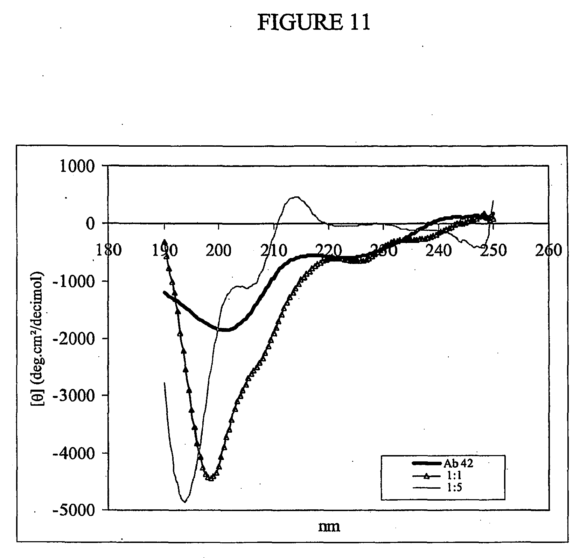

- la figure 11 est une représentation graphique donnant l'élipticité molaire en fonction de la longueur d'onde obtenues par dichroïsme circulaire du peptide bêta Amyloïde 1-42 après réaction avec l'agent II chondroïtine-6-sulfate selon différentes concentrations, Ab42 étant le témoin sans traitement par l'agent II,

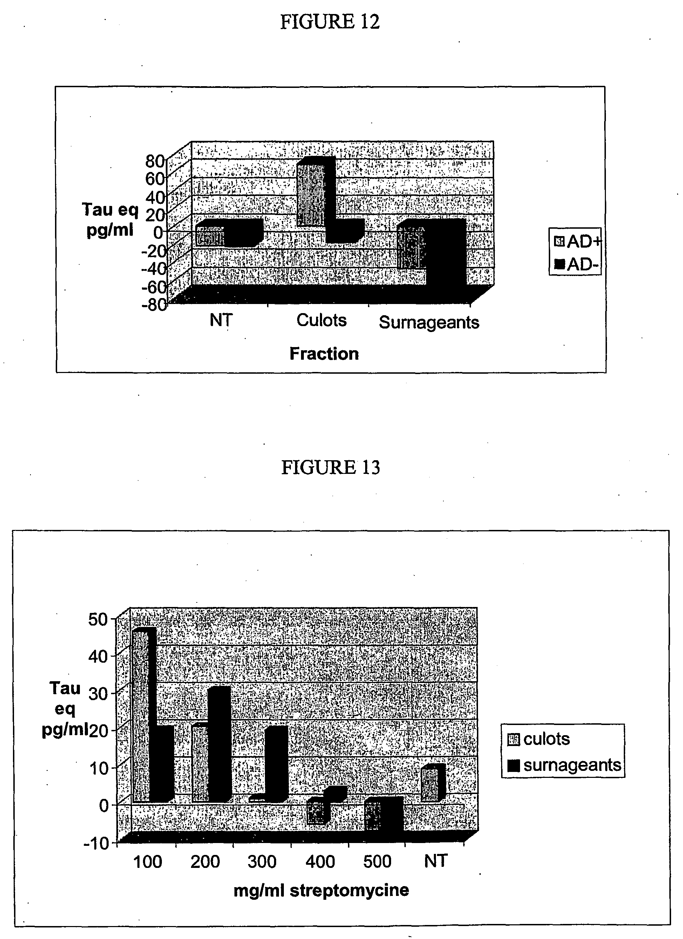

- la figure 12 est une représentation graphique d'un dosage ELISA réalisé à partir d'extraits de cerveaux de patients atteints de la maladie d'Alzheimer (AD+) ou non (AD-), non traités (NT) ou traités par l'agent I, qui est la triéthylènetétramine (TET). La détection de la protéine Tau phosphorylée (en pg/ml, dosé en équivalents (eq) de protéine Tau recombinante) est réalisée dans les fractions culots et surnageants,
- la figure 13 une représentation graphique d'un test ELISA réalisé à partir d'extraits de cerveaux de patients atteints de la maladie d'Alzheimer, non traités (NT) ou traités par différentes concentrations (mg/ml) d'agent I, qui est la streptomycine. La détection de la protéine Tau phosphorylée (eq Tau en pg/ml) est réalisée dans les fractions culots et surnageants,

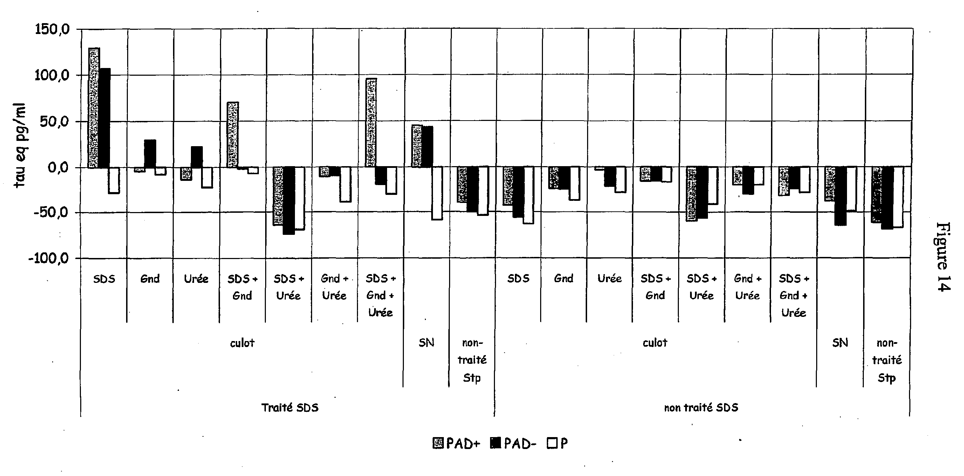

- la figure 14 est une représentation graphique montrant l'effet de différents tampons de reprise sur la détection de la protéine Tau phosphorylée, après traitement ou non par du SDS 1% et précipitation par la streptomycine (500 mg/ml). Les résultats ont été obtenus à partir d'extraits de cerveaux de patients atteints de la maladie d'Alzheimer (PAD+) ou non (PAD-), ou de plasma non surchargé ·(P). Gnd : guanidine ; SN : surnageant; stp: streptomycine,

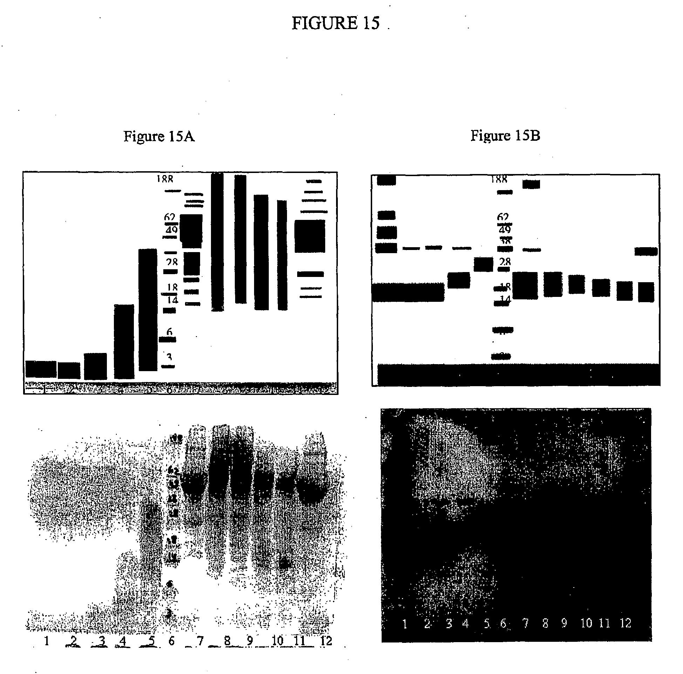

- la figure 15 est une représentation schématique (panel haut) d'un gel d'électrophorèse (panel bas) après coloration par le bleu de Coomassie (figure 15A) et de la radiographie du même gel à la suite d'un Western Blot (figure 15B), obtenu après migration de la protéine SYN dans le plasma après traitement par la TET (pistes 2-5 : culots ; pistes 8-11 : surnageants). Les pistes 1 et 7 correspondent aux culots et surnageants de l'échantillon témoin sans ajout d'agent I, la piste 6 contient des marqueurs de poids moléculaire (188, 62, 49, 38, 28, 18, 14, 6 et 3 kDa )et la piste 12 correspond à la protéine recombinante seule,

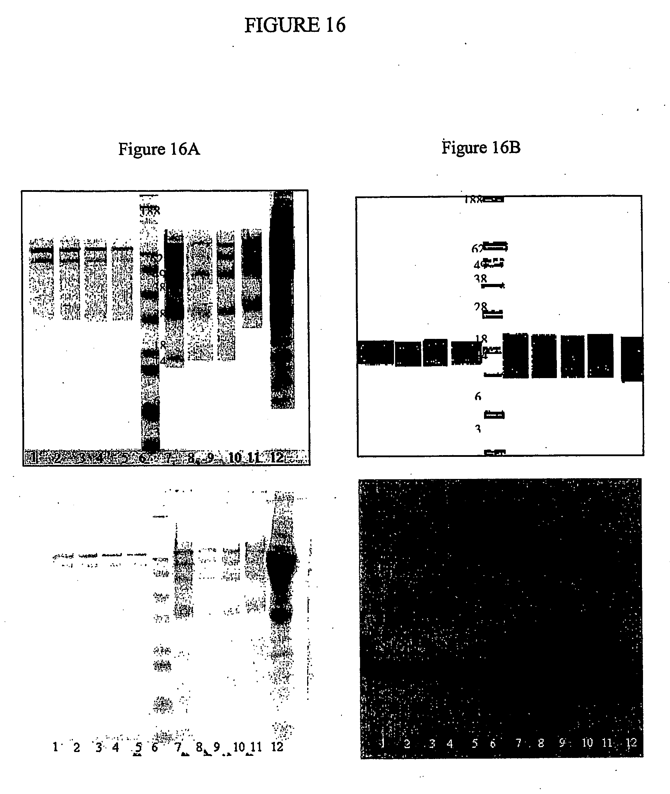

- la figure 16 est une représentation schématique (panel haut) d'un gel d'électrophorèse (panel bas) après coloration par le bleu de Coomassie (figure 16A) et de la radiographie du même gel à la suite d'un Western Blot (figure 16B), obtenu après migration de la protéine SYN dans le plasma après traitement par la streptomycine (pistes 2-5 : culots ; pistes 8-11 : surnageants). Les pistes 1 et 7 correspondent aux culots et surnageants de l'échantillon témoin sans ajout d'agent I , la piste 6 contient des marqueurs de poids moléculaire (188, 62, 49, 38, 28, 18, 14, 6 et 3 kDa) et la piste 12 correspond à la protéine recombinante seule,

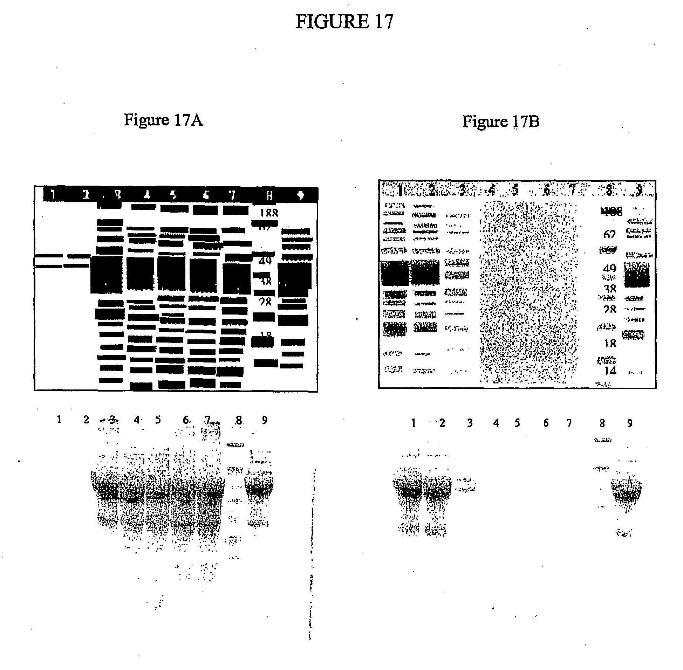

- la figure 17 est une représentation schématique (panel haut) d'un gel d'électrophorèse (panel bas) après coloration par le bleu de Coomassie obtenu après migration de la protéine SYN dans le plasma après traitement par les calixarènes (agent II). La figure 17A correspond à la fraction culots alors que la figure 17B correspond à la fraction surnageants. Les pistes 1 à 7 correspondent à la protéine Syn traitée par différentes concentrations de calixarènes, la piste 8 contient des marqueurs de poids moléculaire (188, 62, 49, 38, 28 et 14 kDa) et la piste 9 correspond à la protéine recombinante non traitée,

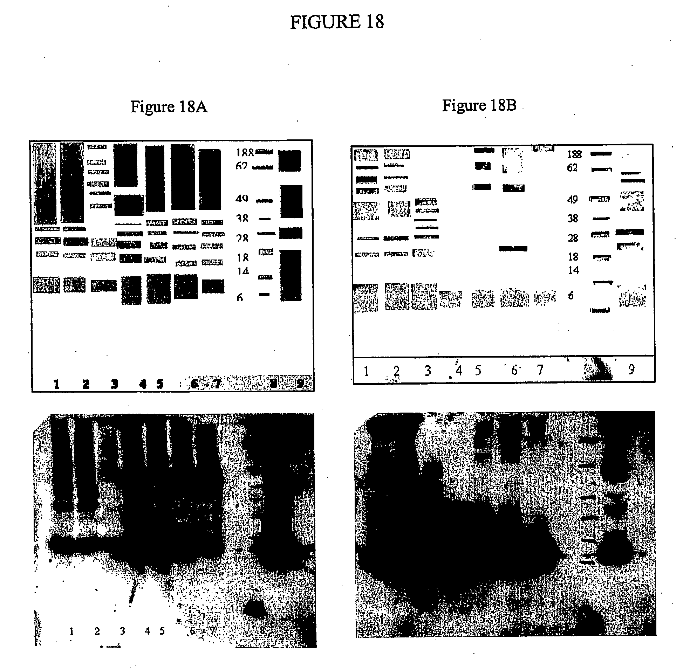

- la figure 18 est une représentation schématique (panel haut) de l'autoradiographie (panel bas) d'un gel d'électrophorèse à la suite d'un Western-blot, obtenu après migration de la protéine SYN dans le plasma après traitement par les calixarènes (agent II). La figure 18A correspond à la fraction culots alors que la figure 18B correspond à la fraction surnageants. Les pistes 1 à 7 correspondent à la protéine Syn traitée par différentes concentrations de calixarènes, la piste 8 contient des marqueurs de poids moléculaire (188, 62, 49, 38, 28, 18, 14 et 6 kDa) et la piste 9 correspond à la protéine recombinante non traitée,

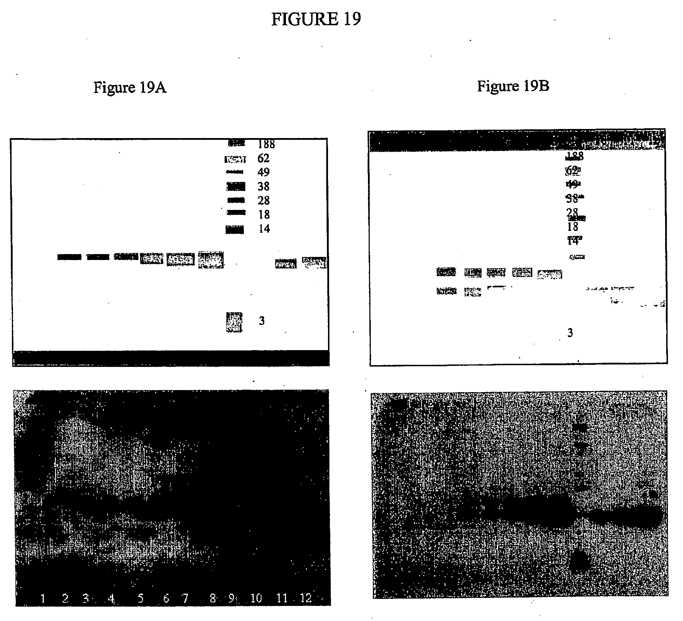

- la figure 19 est une représentation schématique (panel haut) de l'autoradiographie (panel bas) d'un gel d'électrophorèse à la suite d'un Western-blot, obtenu après migration de la protéine SYN dans le plasma après traitement par les calixarènes (agent II). La figure 19A correspond à la fraction culots alors que la figure 19B correspond à la fraction surnageants. Les pistes 1 à 8 correspondent à différentes concentrations de la protéine Syn traitée par les calixarènes, les pistes 10 à 12 correspondent à la protéine Syn non traitée et la la piste 9 contient des marqueurs de poids moléculaire (188, 62, 49, 38, 28, 18, 14 et 3 kDa),

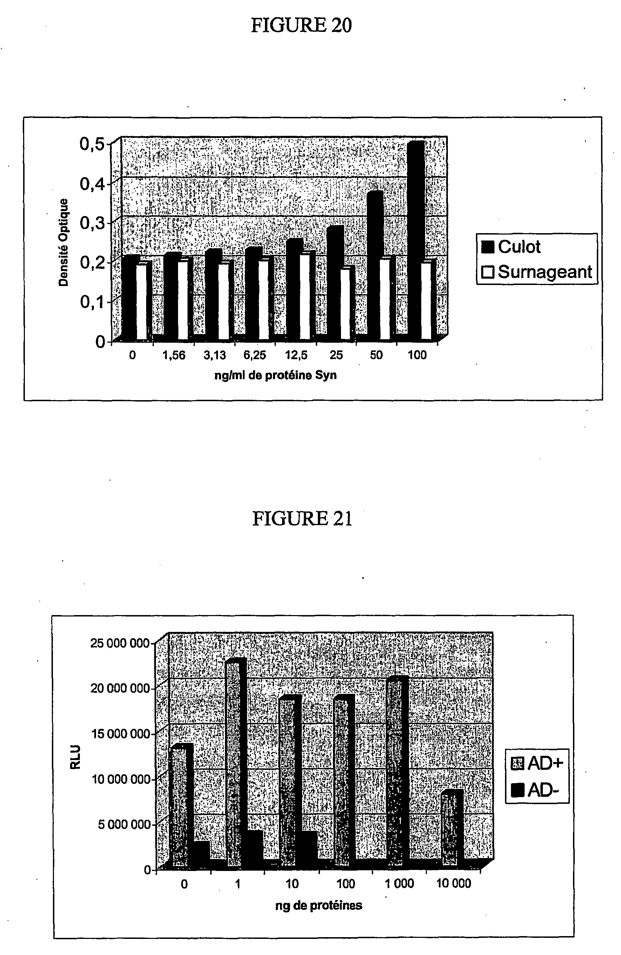

- la figure 20 est une représentation graphique d'un dosage ELISA réalisé à partir d'une gamme de concentrations (ng/ml) de protéine Syn présente dans le plasma, et traitée par les calixarènes (agent II). La densité optique est lue dans les fractions culots et surnageants,
- la figure 21 est une représentation graphique d'un dosage ELISA de type sandwich dans lequel les calixarènes (agent II) sont utilisés dans la phase de capture. Différentes quantités (ng) de protéines issues d'extraits de cerveaux de patients atteints de la maladie d'Alzheimer (AD+) ou non atteints (AD-) sont testées. Les résultats obtenus sont exprimés sous forme d'unité relative de lumière (RLU),

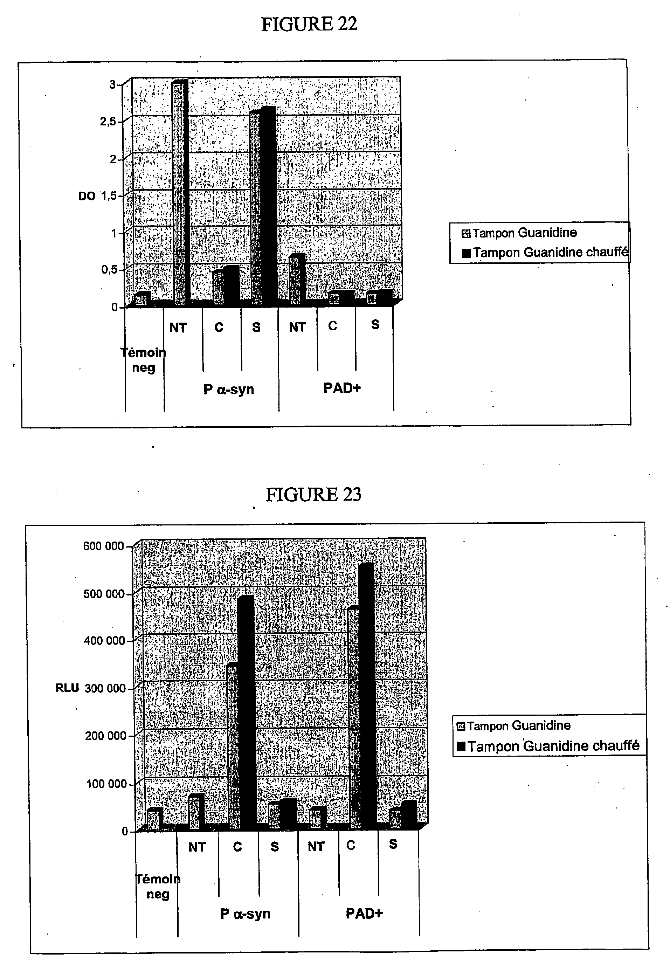

- la figure 22 est une représentation graphique d'un dosage ELISA de type sandwich utilisant un anticorps spécifique en phase de capture. Le dosage est réalisé à partir d'extraits de cerveaux de patients atteints de la maladie d'Alzheimer (PAD+) ou à partir de la protéine alpha-synucléine recombinante (Pα-syn), surchargés dans un pool de plasma humains, et précipités par un agent I, qui est la streptomycine. Après précipitation, les culots ( C ) sont repris dans un tampon guanidine chauffé ou non. Les résultats sont exprimés sous forme de densité optique. NT signifie non traité, C culot et S surnageant,
- la figure 23 est une représentation graphique d'un dosage ELISA de type sandwich après précipitation par l'agent I (streptomycine) et détection par ELISA avec capture par l'agent II (calixarène). Le dosage est réalisé à partir d'extraits de cerveaux de patients atteints de la maladie d'Alzheimer (PAD+) ou à partir de la protéine alpha-synucléine recombinante (Pα-syn), surchargés dans un pool de plasma humains. Après précipitation avec l'agent I, les culots sont repris dans un tampon guanidine chauffé ou non. Les résultats sont exprimés sous forme de densité optique,

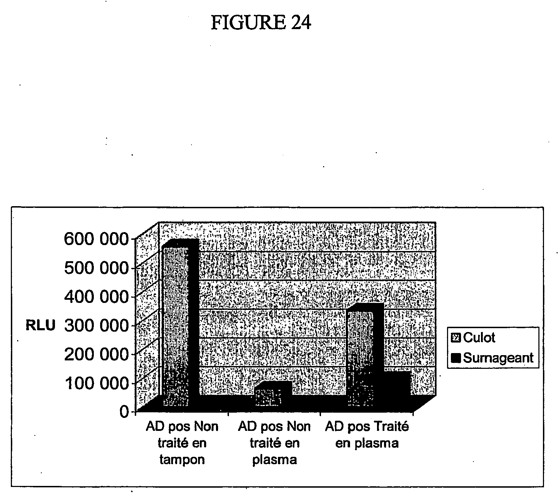

- la figure 24 est une représentation graphique d'un dosage ELISA de la protéine Tau phosphorylée, réalisé à partir d'un extrait de cerveau de patient atteint de la maladie d'Alzheimer (AD pos) surchargé dans un pool de plasmas de patients sains, non traité ou traité par l'agent I, qui est la streptomycine. La phase de capture est constituée par des calixarènes (agent II). Les résultats obtenus dans les fractions culots et surnageants sont exprimés sous forme d'unités relatives de lumière (RLU).

### Exemple 1 : Analyse comparée des séquences peptidiques des protéines associées aux dépôts d'agrégats neurotoxiques dans les maladies neurodégénératives non infectieuses

Nous avons utilisé le logiciel « Mac Vector » pour réaliser un alignement des séquences peptidiques primaires de protéines représentatives des protéines associées à des dépôts d'agrégats neurotoxiques dans les maladies neurodégénératives, à savoir les protéines alpha-synucléine (SEQ ID N°1), précurseur de la protéine amyloide ou APP (SEQ ID N°2), Parkine (SEQ ID N°3) et protéine Tau (SEQ ID N°4). Les acides aminés y sont numérotés au dessus de l'alignement, sur la base de leur coïncidence avec la séquence consensus la plus longue, et les tirets dans la séquence d'une protéine représentent un décalage entre sa séquence peptidique linéaire et l'alignement de motifs communs à l'ensemble des protéines sur la séquence consensus.

Un alignement de type « Clustal W » avec une option d'alignement multiple a été effectué et est présenté dans la figure 1. Les zones d'homologies (acides aminés identiques ou de fonctionnalité chimique équivalente) sont encadrées et définissent des domaines communs à ces différentes protéines dont une propriété commune est de former des agrégats neurotoxiques dans le système nerveux central des maladies neurodégénératives.

Les acides aminés encadrés individuellement sont représentatifs de répétition plus fréquente de certains acides aminés d'intérêt dans la séquence primaire d'une protéine en particulier. Ces répétitions peuvent être réparties différemment selon les protéines, mais constituent une base fonctionnelle plus dense que le consensus, ou bien leur projection dans l'espace peut constituer un domaine de liaison.

Sur la base de nos connaissances originales sur les fonctions impliquées dans les liaisons avec les agents I tels que les composés organiques comprenant au moins deux fonctions guanidinium et/ou pyridinium et/ou ammonium, ou les agents II tels que les calix-arènes de type Calix-6-arène-Sulphonate, nous avons pu identifier les acides aminés portant les fonctions précédemment décrites avec une densité particulière au niveau de ces régions homologues.

On peut ainsi voir sur la figure 1 que, pour se lier aux agents I tels que composés organiques comprenant au moins deux fonctions guanidinium et/ou pyridinium et/ou ammonium, les régions telles que définies ci-après contiennent un minimum de 4 acides aminés choisis parmi D, E, S ou N, dans une séquence peptidique de longueur inférieure ou égale à 20 acides aminés :
i) pour l'alpha Synucléine, par exemple, les régions
   - 124-143 ont 4 résidus D, E, S ou N sur 20 acides aminés,
   - 231-261 ont 10 résidus D, E, S ou N sur 19 acides aminés (les tirets indiquent un décalage de la numérotation basée sur le consensus par rapport à la séquence primaire, ce qui fait que les 20 acides aminés sont numérotés sur un nombre supérieur à leur nombre réel dans l'intervalle de séquence de la protéine) et
   - 232-249 ont même 6 résidus D, E, S ou N sur 12 acides aminés ;
ii) pour l'APP, par exemple, les régions
   - 80-98 ont 5 résidus D, E, S ou N sur 20 acides aminés,
   - 193-216 ont 14 résidus D, E, S ou N sur 17 acides aminés,
   - 225-236 ont 6 résidus D, E, S ou N sur 12 acides aminés,
   - 247-256 ont 9 résidus D, E, S ou N sur 10 acides aminés,
   - 692-702 ont 5 résidus D, E, S ou N sur 11 acides aminés,
   - 713-718 ont 4 résidus D, E, S ou N sur 6 acides aminés ;
iii) pour la Parkine, par exemple, les régions
   - 25-46 ont 8 résidus D, E, S ou N sur 20 acides aminés (du fait des décalages de numérotation avec le consensus),
   - 153-172 ont 8 résidus D, E, S ou N sur 20 acides aminés,
   - 224-240 ont 5 résidus D, E, S ou N sur 12 acides aminés ;
iv) pour la Protéine Tau les régions
   - 31-53 ont 6 résidus D, E, S ou N sur 20 acides aminés,
   - 225-248 ont 12 résidus D, E, S ou N sur 20 acides aminés,
   - 236-247 ont 9 résidus D, E, S ou N sur 12 acides aminés,
   - 289-308 ont 8 résidus D, E, S ou N sur 20 acides aminés,
   - 361-380 ont 8 résidus D, E, S ou N sur 20 acides aminés,
   - 523-542 ont 9 résidus D, E, S ou N sur 20 acides aminés, et
   - 753-764 ont 5 résidus D, E, S ou N sur 12 acides aminés.

On peut également noter qu'une densité de ces acides aminés (D, E, S ou N) supérieure ou égale à 4 sur une séquence peptidique inférieure ou égale à 20 acides aminés peut aussi être retrouvée au moins deux fois sur la séquence complète de la protéine constituant les FPCA. De plus, on constate que ces acides aminés (D, E, S ou N) peuvent être présents globalement au moins 5 fois dans une séquence peptidique de longueur inférieure ou égale à 20 acides aminés et/ou au moins 4 fois dans une séquence peptidique de longueur inférieure ou égale à 16 acides aminés. Enfin, une densité telle que définie précédemment peut aussi être associée avec une zone de plus forte densité pour ces acides aminés (D, E, S ou N), correspondant à au moins 5 dans une séquence peptidique de longueur inférieure ou égale à 12 acides aminés. Une densité de fonctions équivalentes sur une distance spatiale équivalente peut aussi constituer une structure mimétique de celle qui a été définie précédemment dans une structure primaire de protéine.

Il est aussi intéressant de noter que la fréquence de ces acides aminés peut atteindre 32 sur 49 acides aminés contigus sur la séquence peptidique de l'APP, sans pour autant exclure d'autres régions de fixation à distance de cet enchaînement de 49 résidus (région 225-275).

Par ailleurs, on peut également voir sur la figure 1 que, pour se lier aux agents II tels que molécule macrocyclique de type calix-arènes, les régions telles que définies ci-après contiennent un minimum de 3 acides aminés basiques -de préférence l'arginine (R), la lysine (K), l'histidine (H) ou la glutamine (Q)- dans une séquence peptidique de longueur inférieure ou égale à douze, voire quinze, acides aminés :
i) pour l'alpha Synucléine les régions
   - 106-120 ont 5 résidus K, H, Q ou R sur 15 acides aminés,
   - 128-142 ont 4 résidus K, H, Q ou R sur 15 acides aminés,
   - 159-163 ont 3 résidus K, H, Q ou R sur 15 acides aminés ;
ii) pour l'APP les régions
   - 90-104 ont 7 résidus K, H, Q ou R sur 15 acides aminés,
   - 105-116 ont 4 résidus K, H, Q ou R sur 12 acides aminés,
   - 132-143 ont 5 résidus K, H, Q ou R sur 12 acides aminés,
   - 153-163 ont 3 résidus K, H, Q ou R sur 12 acides aminés,
   - 697-707 ont 5 résidus K, H, Q ou R sur 11 acides aminés ;
iii) pour la Parkine les régions
   - 83-100 ont 7 résidus K, H, Q ou R sur 15 acides aminés,
   - 182-195 ont 6 résidus K, H, Q ou R sur 12 acides aminés ;
iv) pour la Protéine Tau les régions
   - 101-108 ont 4 résidus K, H, Q ou R sur 8 acides aminés,
   - 135-147 ont 4 résidus K, H, Q ou R sur 13 acides aminés,
   - 178-183 ont 3 résidus K, H, Q ou R sur 6 acides aminés,
   - 197-208 ont 5 résidus K, H, Q ou R sur 1 acides aminés.

On peut également noter qu'une densité de trois charges positives équivalentes, sur une distance spatiale équivalente, peut aussi constituer une structure mimétique de celle qui a été définie précédemment dans une structure primaire de protéine. Ce peut-être, par exemple, une conformation qui projette ces cations ou leurs équivalents fonctionnels dans un espace tridimensionnel équivalent à une séquence de douze, voire quinze, acides aminés sur une portion de cet espace représentant une hélice alpha constituée d'acides aminés.

### Exemple 2 : Détection de la protéine Tau par un procédé utilisant un agent I

### 2.1 Technique d'analyse

L'analyse est réalisée selon la technique de Western Blotting (Laemmli, UK 1970, Nature, 227 : 680-685).

Les échantillons à analyser sont dénaturés en tampon SDS (125 mM Tris HCl pH 6,8, 20% glycérol, 4 % SDS, 0,02% bleu de bromophénol) (50/50 v/v) à 100°C pendant 5 minutes. Ceux-ci sont ensuite déposés sur un gel d'électrophorèse unidimensionnel de polyacrylamide 12% en présence de sodium dodecyl sulfate (SDS-PAGE).

Après migration, les protéines sont transférées sur membrane de nitrocellulose et immunoblottées à 4°C pendant une nuit avec un anticorps monoclonal spécifique de la protéine Tau (anticorps Tau-1 (Chemicon)).

L'anticorps secondaire de détection est un anticorps de chèvre reconnaissant les chaînes lourdes et légères des immunoglobulines G de souris conjugué à la peroxydase de Raifort.

La membrane est lavée entre chaque étape en tampon phosphate salin (PBS) avec puis sans Tween 20 (0,05% p/v).

Les signaux sont détectés par chimioluminescence avec le kit super signal (Pierce ) et visualisés sur un film radiographique (Pierce).

### 2.2 Détection en présence de l'agent I

Les essais sont réalisés avec 1 µg de protéine Tau recombinante fusionnée à une queue de 6 histidines (Calbiochem) et avec, comme agent I, soit de la streptomycine, sous forme de sulfate, soit de la triéthylènetétramine ou TET, soit de la rifampicine ou soit de l'isoniazide, avec un ratio quantité d'agent I (µg) sur quantité de protéines totales (µg) égal à 9/1 ou 35/1.

Une fois que la protéine recombinante et l'agent I sont mis en contact, le mélange est incubé pendant 30 minutes à 37°C.

Les échantillons sont ensuite centrifugés pendant 10 minutes à 13000 rpm. Les culots sont récupérés et après dénaturation, ils sont analysés par la technique du Western Blotting.

Les résultats sont représentés sur les figures 2 et 3 telles que définies ci-après :
- la figure 2 est une représentation schématique (figure 2A) de la radiographie d'un gel d'électrophorèse (figure 2B) du Western Blotting, obtenu après migration de la protéine Tau traitée par la streptomycine (pistes 3 et 4), ou par la TET (pistes 5 et 6), la piste 1 correspondant à la piste des marqueurs de poids moléculaire et la piste 2 correspondant à l'échantillon témoin sans traitement par l'agent I, et
- la figure 3 est une représentation schématique (figure 3A) de la radiographie d'un gel d'électrophorèse (figure 3B) du Western Blotting, obtenu après migration de la protéine Tau traitée par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I.

Les concentrations en agent I par rapport aux pistes d'électrophorèse sont telles qu'indiquées dans les tableaux 1 et 2 ci-dessous.

**Tableau 1**

| **piste** | **Echantillon déposé** | **fraction** |
|---|---|---|
| 1 | Marqueur de poids moléculaire | Pas de fractionnement |
| 2 | Tau recombinante non traitée, témoin positif de western blotting | Pas de fractionnement |
| 3 | µg streptomycine / µg Tau recombinante : 9/1 | Culot |
| 4 | µg streptomycine / µg Tau recombinante : 35/1 | Culot |
| 5 | µg TET / µg Tau recombinante : 9/1 | Culot |
| 6 | µg TET / µg Tau recombinante: 35/1 | Culot |

**Tableau 2**

| **piste** | **Echantillon déposé** | **fraction** |
|---|---|---|
| 1 | Marqueur de poids moléculaire | Pas de fractionnement |
| 2 | Tau recombinante non traitée, témoin positif de western blotting | Pas de fractionnement |
| 3 | µg rifampicine / µg Tau recombinante: 2/1 | Culot |
| 4 | µg rifampicine / µg Tau recombinante: 9/1 | Culot |
| 5 | µg rifampicine / µg Tau recombinante: 35/1 | Culot |
| 6 | Marqueur de poids moléculaire | Pas de fractionnement |
| 7 | Tau recombinante non traitée, témoin positif de western blotting | Pas de fractionnement |
| 8 | µg isoniaside / µg Tau recombinante: 2/1 | Culot |
| 9 | µg isoniaside / µg Tau recombinante: 9/1 | Culot |
| 10 | µg isoniaside / µg Tau recombinante: 35/1 | Culot |

Les résultats montrent que la protéine Tau et ses formes digérées sont retrouvées dans le culot après traitement avec l'agent I, que ce soit la streptomycine, la TET, la rifampicine ou l'isoniazide. Leur présence dans le culot de sédimentation atteste de leur concentration à partir du volume d'échantillon testé, ce qui ne se produit pas en absence d'agent I. Ces molécules induisent donc une précipitation de la protéine Tau après une incubation de 30 minutes à 37°C et une centrifugation simple.

### Exemple 3 : Détection de la protéine Tau par un procédé utilisant un agent II (procédé non compris dans l'invention)

### 3.1 Technique d'analyse

Elle est identique à celle décrite dans le point 2.1 ci-dessus.

### 3.2 Détection réalisée en présence du calix-arène p-sulphonato-3,7-(2-amino-éthyloxy)-calix-[6]-arène (dénommé C6S) à titre d'agent II

Les essais sont réalisés comme indiqué dans le point 2.2 ci-dessus, à ceci près qu'on a utilisé du C6S préparé comme indiqué dans la demande de brevet WO2004/059322, avec une gamme de concentration de 0,5 mM à 60 mM.

Les résultats sont représentés sur la figure 4 qui est une représentation schématique (figure 4A) de la radiographie d'un gel d'électrophorèse (figure 4B) du Western Blotting, obtenu après migration de la protéine Tau traitée par le calix-arène p-sulfonato-3,7-(2-aminoéthyloxy)-calix-[6]-arène comme agent II, la fraction récupérée étant soit le culot (pistes 2 à 9), soit le surnageant (pistes 12 à 20), les pistes 1 et 21 correspondant à l'échantillon témoin sans traitement par le calix-arène et la piste 11 correspondant à la piste des marqueurs de poids moléculaire.

Les concentrations en agent II par rapport aux pistes d'électrophorèses sont telles qu'indiquées dans le tableau 3 ci-dessous.

**Tableau 3**

| **piste** | **Echantillon déposé** | **fraction** |
|---|---|---|
| 1 | Tau recombinante non traitée, témoin positif de western blotting | Pas de fractionnement |
| 2 | Tau recombinante + 0,5mM final de C6S | Culot |
| 3 | Tau recombinante + 2,5mM final de C6S | Culot |
| 4 | Tau recombinante + 5mM final de C6S | Culot |
| 5 | Tau recombinante + 10mM final de C6S | Culot |
| 6 | Tau recombinante + 20mM final de C6S | Culot |
| 7 | Tau recombinante + 30mM final de C6S | Culot |
| 8 | Tau recombinante + 40mM final de C6S | Culot |
| 9 | Tau recombinante + 50mM final de C6S | Culot |
| 10 | Tau recombinante avec 60mM final de C6S | Culot |
| 11 | Marqueur de poids moléculaire | Pas de fractionnement |
| 12 | Tau recombinante + 60mM final de C6S | Surnageant |
| 13 | Tau recombinante + 50mM final de C6S | Surnageant |
| 14 | Tau recombinante + 40mM final de C6S | Surnageant |
| 15 | Tau recombinante + 30mM final de C6S | Surnageant |
| 16 | Tau recombinante + 20mM final de C6S | Surnageant |
| 17 | Tau recombinante + 10mM final de C6S | Surnageant |
| 18 | Tau recombinante + 5mM final de C6S | Surnageant |
| 19 | Tau recombinante + 2,5mM final de C6S | Surnageant |
| 20 | Tau recombinante + 0,5mM final de C6S | Surnageant |
| 21 | Tau recombinante non traitée, témoin positif de western blotting | Pas de fractionnement |

Ces résultats montrent que la protéine Tau et ses formes digérées sont retrouvées dans le culot après traitement avec le C6S et, qu'en l'absence d'agent II, aucune concentration ni séparation de la protéine Tau n'est observée. Ces molécules induisent donc une capture de la protéine Tau après une incubation de 30 minutes à 37°C et la sédimentent par une centrifugation simple. De plus, un optimum de précipitation est constaté de 0,5 mM à 40 mM.

### Exemple 4 : Détection des peptides bêta-amyloïdes par un procédé utilisant un agent I ou un agent II (procédé non compris dans l'invention)

On a repris le mode opératoire indiqué dans les exemples 2 et 3 ci-dessus, à ceci près qu'on a utilisé :
- 10 µg de peptide bêta-amyloïde synthétique de 40 acides aminés de longueur (peptide bêta-A 1-40 ; SEQ ID N°5) ou 10 µg de peptide bêta-amyloïde synthétique de 42 acides aminés de longueur (peptide bêta-A 1-42 ; SEQ ID N°6),
- un anticorps monoclonal spécifique des peptides Amyloïdes (anticorps 6E10 (Sigma)),
- comme agent 1 la streptomycine et la TET, avec un ratio quantité d'agent 1 (µg) sur quantité de protéines totales (µg) égal à 35/1, ou bien la rifampicine et l'isoniazide, avec un ratio quantité d'agent 1 (µg) sur quantité de protéines totales (µg) égal à 2/1, 9/1 ou 35/1, et
- comme agent II le C6S à la concentration 1 mM finale.

On a effectué le Western Blot avec les culots de centrifugation qui ont été analysés comme décrit dans l'exemple 2.

Les résultats sont représentés sur les figures 5 à 8 telles que définies ci-après :
- la figure 5 est une représentation schématique (figure 5A) de la radiographie d'un gel d'électrophorèse (figure 5B) du Western Blotting, obtenu après migration du peptide peptide bêta-Amyloïde 1-40 traité par le C6S (piste 2), par la streptomycine (piste 3) ou par la TET (piste 4), la piste 1 correspondant à l'échantillon témoin sans traitement par l'agent I ou II et la piste 5 correspondant à la piste des marqueurs de poids moléculaire,
- la figure 6 est une représentation schématique (figure 6A) de la radiographie d'un gel d'électrophorèse (figure 6B) du Western Blotting, obtenu après migration du peptide bêta-Amyloïde 1-40 traité par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I,
- la figure 7 est une représentation schématique (figure 7A) de la radiographie d'un gel d'électrophorèse (figure 7B) du Western Blotting, obtenu après migration du peptide peptide bêta-Amyloïde 1-42 traité par le C6S (piste 2), par la streptomycine (piste 3) ou par la TET (piste 4), la piste 1 correspondant à l'échantillon témoin sans traitement par l'agent I ou II et la piste 5 correspondant à la piste des marqueurs de poids moléculaire,
- la figure 8 est une représentation schématique (figure 8A) de la radiographie d'un gel d'électrophorèse (figure 8B) du Western Blotting, obtenu après migration du peptide bêta-Amyloïde 1-42 traité par un agent I qui est soit la rifampicine (pistes 3, 4 et 5), soit l'isoniazide (pistes 8, 9 et 10), les pistes 1 et 6 correspondant aux pistes des marqueurs de poids moléculaire et les pistes 2 et 7 correspondant à l'échantillon témoin sans traitement par l'agent I.

Les concentrations en agent I ou II par rapport aux pistes d'électrophorèse sont telles qu'indiquées dans les tableaux 4 et 5 ci-dessous, correspondant aux figures 5 et 7 (tableau 4) et aux figures 6 et 8 (tableau 5).

**Tableau 4**

| **piste** | **Echantillon déposé** | **fraction** |
|---|---|---|
| 1 | Peptide non traité, témoin positif de Western blotting | Pas de fractionnement |
| 2 | Peptide traité + 1mM final de C6 monoacide | Culot |
| 3 | µg streptomycine / µg Peptide: 35/1 | Culot |
| 4 | µg TET/µg Peptide: 35/1 | Culot |
| 5 | Marqueur de poids moléculaire | Pas de fractionnement |

**Tableau 5**

| **piste** | **Echantillon déposé** | **fraction** |
|---|---|---|
| 1 | Marqueur de poids moléculaire | Pas de fractionnement |
| 2 | Peptide non traité, témoin positif de Western blotting | Pas de fractionnement |
| 3 | µg rifampicine / µg Peptide: 2/1 | Culot |
| 4 | µg rifampicine / µg Peptide: 9/1 | Culot |
| 5 | µg rifampicine / µg Peptide: 35/1 | Culot |
| 6 | Marqueur de poids moléculaire | Pas de fractionnement |
| 7 | Peptide non traité, témoin positif de Western blotting | Pas de fractionnement |
| 8 | µg isoniazide / µg Peptide: 2/1 | Culot |
| 9 | µg isoniazide / µg Peptide: 9/1 | Culot |
| 10 | µg isoniazide / µg Peptide: 35/1 | Culot |

Comme précédemment, les résultats montrent que les peptides bêta-Amyloïde et leurs oligomères sont retrouvés dans le culot après traitement avec l'agent I ou l'agent II. Ces molécules induisent donc une précipitation des peptides après une incubation de 30 minutes à 37°C et une centrifugation simple.

De plus, comme le montre la figure 7, piste 1, les oligomères de haut poids moléculaire (multimères), non capturés habituellement par les agents protéiques tels qu'anticorps classiquement utilisés, sont capturés et précipités avec l'agent II, ce qui prouve leur efficacité sur ces formes multimérisées. L'agent II permet ainsi de récupérer tant les formes monomériques qu'oligomériques sans les réticuler (différentes bandes observées en migration électrophorétique).

De même, comme le montre la figure 8, piste 5, les oligomères de haut poids moléculaire (multimères), non réticulés habituellement par les agents protéiques tels qu'anticorps classiquement utilisés, sont réticulés et précipités avec l'agent I, ce qui prouve leur efficacité sur ces formes multimérisées. Par ailleurs, l'agent I permet aussi de réticuler toutes les formes monomériques et oligomériques, ce qui est mis en évidence par la migration électrophorétique de l'ensemble des fractions antigéniques bêta amyloïde dans une zone relativement homogène (bande épaisse et traînée ou « smear » ) de haut poids moléculaire.

### Exemple 5 : Interaction du peptide bêta-Amyloïde 1-42 avec le para-sulfonato-calix[4]arène

### 5.1. Technique d'analyse

L'analyse est menée par dichroisme circulaire.

Le peptide bêta-Amyloïde à analyser tel que défini précédemment, également appelé Aβ₄₂, est dissous extemporanément en concentration mère 2,2.10⁻⁴M dans une solution de TriFluoroEthanol (TFE) à 20% dans l'eau pour structurer les peptides β-Amyloïde. Les *para*-sulfonato-calix[n]arènes, préparés comme décrit dans Da Silva, E. et al., 2003, Tetrahedron, 59(37) : 7357-7364, sont dissous à 100µM dans du tampon phosphate 50mM. Les échantillons sont préparés afin d'obtenir une concentration finale de 10µM de peptide bêta-Amyloïde dans du tampon phosphate, soit pour un volume final de 500µL. Les rapports de concentration peptide/calixarène sont variables de 1/0 à 1/5.

Un témoin β-amyloïde sans *para*-sulfonato-calix[n]arène est effectué : 22,5µL de solution mère Aβ₄₂ 2,2.10⁻⁴M (5 nmoles) dans TFE 20%, 477,5µL de tampon phosphate 50mM.

Les échantillons sont analysés par dichroïsme circulaire entre 180 et 260 nm

### 5.2. Interaction du peptide Aβ₄₂ avec le para-sulfonato-calix[4]arène

Les essais sont réalisés pour des rapports de concentration peptide Aβ₄₂/calixarène 1/1, 1/2, et 1/5, comme indiqué dans le tableau 6 ci-après. Le témoin est un échantillon de peptide Aβ₄₂ sans calixarène (1/0). Les solutions sont préparées pour un volume final de 500µL. Le peptide Aβ₄₂ a une masse molaire de 4514g/mole, le *para*-sulfonato-calix[4]arène a une masse molaire de 744g/mole.

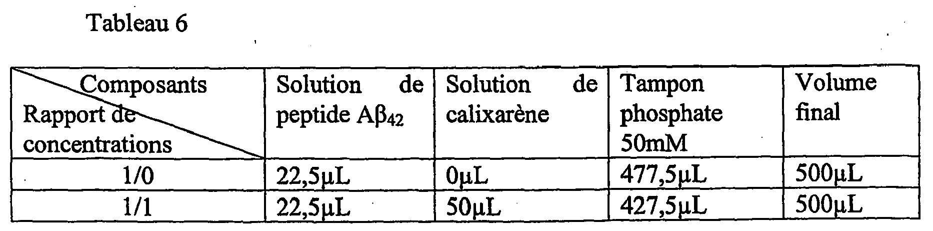

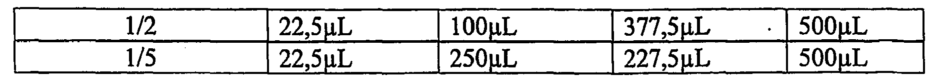

Les résultats sont présentés dans la figure 9 qui est un graphe donnant l'élipticité molaire en fonction de la longueur d'onde obtenues pour le témoin sans calix-arène (Ab42) et pour les rapports de concentration 1/1, 1/2, 1/5.

Les courbes sur la figure 9 mettent en évidence que le peptide initialement en hélice α, adopte en présence du *para*-sulfonato-calix[4]arène les trois structures, hélice α (210 nm), feuillet β (220 nm) et apériodique (200 nm), quelle que soit la concentration de calix[4]arène utilisée, soit à faible concentration (1/1) ou à forte concentration (1/5). La structure en hélice α représente plus de 37% des structures visibles, les feuillets β plus de 26% et enfin la structure apériodique est présente à 38%.

Nous constatons qu'au rapport de concentration 1/1, l'ellipticité molaire des structures augmente par rapport à 1/2. En revanche, le spectre de concentration en rapport 1/5 du peptide Aβ₄₂ se rapproche de celui de 1/1 vers les longueurs d'ondes voisines de 210 nm et se rapproche du 1/2 au-delà.

Ces variations mettent en évidence l'interaction moléculaire entre l'agent II et le peptide bêta-Amyloïde par les modifications structurales significatives observées sur le spectre.

### Exemple 6 : Etude de complexation du peptide β-amyloïde 1-40 avec les para-sulfonato-calix[n]arènes par spectrométrie de masse en mode électrospray

### 6.1. Technique d'analyse

Le peptide β-Amyloïde (également appelé Aβ₄₀) et les différents *para*-sulfonato-calix[n]arènes (n=4, 6, 8), préparés comme décrit par Da Silva, E. et al., 2003, *supra*, sont solubilisés à une concentration de 100µM dans du tampon acétate d'ammonium 10mM. La solution de peptide β-amyloïde à tester est mélangée extemporanément à la solution de para-sulfonato-calix[n]arènes. Les échantillons sont injectés directement en spectrométrie de masse en mode électrospray.

### 6.2. Etude de complexation du peptide Aβ₄₀ avec les différents para-sulfonato-calix[n]arènes

Une solution mère de peptide Aβ₄₀ 10µM (Mw=4430g/mol) dans un tampon acétate d'ammonium 10mM est préparée. Les différents *para*-sulfonato-calix[n]arènes (n=4 :SC4 Mw=744g/mol; n=6 :SC6 Mw=1116g/mol ; n=8 :SC8 Mw=1488g/mol) sont solubilisés à 100µM dans un tampon acétate d'ammonium 10mM.

100µL de la solution de peptide Aβ₄₀ 100µM sont mélangés à 100µL d'une solution de *para*-sulfonato-calix[n]arène 100µM. Les échantillons sont injectés extemporanément en spectrométrie de masse en mode électrospray.

Les résultats obtenus sont présentés dans la figure 10 qui est une représentation graphique donnant l'intensité des pics de complexation cps (coup par seconde) obtenue par spectrométrie de masse en mode électrospray du peptide bêta Amyloïde 1-40 après réaction avec les agents II: para-sulfonato-calix[4]arène (SC4), para-sulfonato-calix[6]arène (SC6), para-sulfonato-calix[8]arène (SC8), Ab40 étant le témoin sans traitement par l'agent II.

Ces résultats montrent des pics caractéristiques de la complexation des *para-*sulfonato-calix[n]arènes avec le peptide Aβ40. On peut noter que le témoin est présent en trop large excès, ce qui explique le pic.

### Exemple 7 : Interaction du peptide bêta-Amyloïde 1-42 avec le chondroïtine-6-sulfate

On a repris le mode opératoire décrit dans l'exemple 5 ci-dessus, à ceci près qu'on a utilisé comme agent II le chondroïtine-6-sulfate (Sigma).

Les résultats sont donnés sur la figure 11 qui est une représentation graphique donnant l'élipticité molaire en fonction de la longueur d'onde pour le témoin sans calix-arène (Ab42) et pour les rapports de concentration 1/1 et 1/5.

Les résultats obtenus dans la figure 11 montrent que le spectre d'Aβ₄₂ présente deux minima négatifs, le premier se situe à 201 nm et le deuxième à 226 nm. Ce peptide β-Amyloïde synthétique adopte une structure en hélice α avec une partie en structure apériodique.

En présence de Chondroïtine-6-sulfate, à concentration égale à celle d'Aβ₄₂, la structure du peptide change. Trois structures sont présentes à des pourcentages similaires. En effet, l'hélice α (à 210 nm) est présente à plus de 37%, la structure apériodique ( à 200 nm) est supérieure à 26%. Enfin, le feuillet β apparaît à 220 nm.

En augmentant la concentration de chondroïtine-6-sulfate de cinq fois supérieure à celle du peptide Aβ₄₂, la conformation secondaire de ce dernier change pour adopter une structure essentiellement apériodique.

En conclusion, en présence de Chondroïtine-6-sulfate à concentration égale à celle du peptide amyloïde, des feuillets β apparaissent alors qu'à forte concentration de chondroïtine-6-sulfate cette conformation est remplacée par une structure apériodique.

Ces variations mettent en évidence l'interaction moléculaire entre l'agent II et le peptide bêta-amyloïde par les modifications structurales significatives observées sur le spectre.

### Exemple 8 : Détection de la protéine Tau phosphorylée par un procédé utilisant un agent précipitant I

### 8.1 Technique d'analyse

L'analyse est réalisée selon la technique immunoenzymatique classique de type « ELISA sandwich » comme décrite par Kohnken and coll (Neuroscience Letters 287 (2000) 187-190).
Le test Elisa PhosphoTau repose sur l'utilisation de microplaques Dynex sur lesquelles sont fixés 3 µg/ml d'anticorps de chèvre anti-Fc de souris (Pierce) dans un tampon 25 mM KHPO4, (pH 7,2), 140 mM NaCl, 1 mM EDTA, 2mM NaN3 durant trois heures à 24°C. Les puits sont ensuite passives avec un tampon TBS (25 mM Tris-HCl, pH 7,5, 140 mM NaCl) Caséine (1%) durant 1h à 24°C puis incubés 2h à 24°C avec 3 µg/ml d'anticorps Tau1 (Chemicon) et CP27 (AppliedNeuroSolutions) dilués en tampon TBS
Caséine.
Standards et extraits de cerveaux sont dilués dans un pool de plasmas sains pour ces tests et déposés à raison de 80 µL par puits en double. L'anticorps de détection CP9 (AppliedNeuroSolutions, US) est dilué dans un tampon contenant 200 µg/ml d'albumine humaine, 22 µg/ml d'IgG humaine et 0,15 µg/ml d'IgM humaines dans un tampon bicarbonate Krebs-Ringer. 20 µl de ce mélange sont ajoutés dans chaque puits et incubés 66 heures à 24°C. Afin de reconnaître l'anticorps CP9 fixé, un anticorps de chèvre (F(ab')₂) biotinylé anti IgM de souris (Accurate) à 0,2 µg/ml en tampon TBS caséine contenant 1% de sérum humain normal (Biocell) est ajouté durant 2h à 24°C. La détection de l'anticorps biotinylé est assuré avec 0,4 µg/ml d'un conjugué streptavidine-peroxydase (Pierce) dilué en tampon TBS caséine et incubé durant 45 minutes à 24°C. Un réactif Lumiglo (Kirkegaard and Perry laboratories, US) est ajouté dans chaque puits et la chemiluminescence lue dans un luminomètre (Berthold, Centro LB 960). Les données obtenues sont sous forme d'unité relative de lumière (RLU), les concentrations en pg/ml, dosées en équivalents de tau recombinante, étant générées en reportant les RLU des échantillons sur la courbe des standards (RLU en fonction de la concentration en pg/ml).
Entre chaque étape décrite précédemment les puits sont lavés avec du tampon TBS contenant 0,1% de Tween20.

### 8.2 Détection en présence de l'agent I, le TET

Les essais sont réalisés avec 25 ng d'extraits de cerveaux et avec, comme agent I de la triéthylènetétramine ou TET à 100 mg/ml.
Une fois que l'échantillon et l'agent I sont mis en contact, le mélange est incubé pendant 60 minutes à 37°C.
Les échantillons sont ensuite centrifugés pendant 10 minutes à 13000 rpm. Les culots sont récupérés et les culots sont séparés des surnageants. Toutes les fractions sont conservées. Les culots sont repris avec 250 µl de tampon tris-maléate (sauf indication contraire). Les échantillons sont alors prêts pour être déposés sur plaques.

La figure 12 résume les résultats obtenus sur des extraits de cerveaux de patients Alzheimer (AD+) ou non (AD-) surchargés (« spiking ») dans du plasma. Les résultats ont été générés en absence (NT) ou après traitement par le TET à 100 mg/ml et analyse des deux fractions culot et surnageant.
Les résultats montrent que la protéine Tau phosphorylée est retrouvée dans le culot après traitement avec l'agent I, le TET. Sa présence dans le culot de sédimentation atteste de sa concentration par agrégation à partir du volume d'échantillon testé, ce qui ne se produit pas en absence d'agent I. Ces molécules induisent donc une précipitation de la protéine recombinante Tau après une incubation de 60 minutes à 37°C et une centrifugation simple ; la protéine est alors disponible pour une détection par une technique Elisa utilisable en routine dans un laboratoire clinique.

### 8.3 Détection en présence de l'agent I, 1a streptomycine

Les essais sont réalisés avec 25 ng d'extraits de cerveaux et avec, comme agent I, de la streptomycine, sous forme de sulfate, à différentes concentrations.
Une fois que l'échantillon et l'agent I sont mis en contact, le mélange est incubé pendant 60 minutes à 37°C.
Les échantillons sont ensuite centrifugés pendant 10 minutes à 13000 rpm. Les culots sont récupérés et les culots sont séparés des surnageants. Toutes les fractions sont conservées. Les culots sont repris avec 250 µl de tampon tris-maléate (sauf indication contraire). Les échantillons sont alors prêts pour être déposés sur plaques.

La figure 13 résume les résultats obtenus sur des extraits de cerveaux de patients Alzheimer (AD+) surchargés dans du plasma. Les résultats ont été générés en absence (NT) ou après traitement par la streptomycine à différentes concentrations et analyse des deux fractions culot et surnageant.
Les résultats montrent que la protéine Tau phosphorylée est retrouvée dans le culot après traitement avec l'agent I, la streptomycine à faible concentration. Sa présence dans le culot de sédimentation atteste de sa concentration par agrégation à partir du volume d'échantillon testé, ce qui se traduit par une augmentation de la détection comparé à l'analyse du même échantillon en absence de traitement par l'agent I. Ces molécules induisent donc une précipitation de la protéine recombinante Tau après une incubation de 60 minutes à 37°C et une centrifugation simple ; la protéine est alors disponible pour une détection par une technique Elisa utilisable en routine dans un laboratoire clinique. Toutefois on voit que la précipitation n'est pas totale puisqu'il subsiste de la protéine dans le surnageant. L'augmentation de 1a quantité d'agent I permet de supprimer la présence de la protéine dans le surnageant pour une concentration de 500 mg/ml. Dans ce cas la protéine précipitée n'est plus détectable dans le culot par une technique Elisa. Des analyses par Western Blot (données non communiquées) ont permis de vérifier que la protéine est bien présente sous forme agrégée. Pour détecter ladite forme agrégée avec un anticorps, un tampon de reprise adapté est nécessaire pour réexposer les épitopes de chaque protéine (voir point 8.4).

### 8.4 Détection en présence de l'agent I. la streptomycine : choix d'un tampon de reprise

Les essais sont réalisés avec 25 ng d'extraits de cerveaux et avec, comme agent I, de la streptomycine, sous forme de sulfate, à 500 mg/ml.
Les échantillons sont préalablement traités ou non par du SDS (1% final).
Une fois que l'échantillon et l'agent I sont mis en contact, en présence ou absence de SDS, le mélange est incubé pendant 60 minutes à 37°C.
Les échantillons sont ensuite centrifugés pendant 10 minutes à 13000 rpm. Les culots sont récupérés et sont séparés des surnageants. Toutes les fractions sont conservées. Les culots sont repris avec 250 µl de différentes solutions :
- SDS 1%
- Guanidine HCl 0,1M (Gnd)
- Urée 1M
- SDS 1% + Guanidine HCl 0,1M (Gnd)
- SDS 1% + Urée 1M
- Guanidine HCl 0,1 M (Gnd)+ Urée 1M
- SDS 1% + Guanidine HCl 0,1M (Gnd)+ Urée 1M
Les échantillons sont alors prêts pour être déposés sur plaques
Les résultats (figure 14) montrent qu'en absence de SDS lors de l'étape de précipitation, la détection de la protéine n'est pas optimale.
Quand la précipitation est réalisée en présence de SDS, les résultats diffèrent selon le type de tampon de remise en suspension utilisé. Une fois encore la présence de SDS dans ce tampon de reprise est indispensable. Les deux meilleures combinaisons sont SDS-Guanidine et, plus encore, SDS-Guanidine-Urée qui permet une meilleure intensité de détection. L'échantillon Alzheimer positif (PAD+) est alors parfaitement dissocié de l'échantillon Alzheimer négatif (PAD-) ou du plasma non surchargé (P). Sa présence dans le culot de sédimentation atteste ainsi de sa concentration par agrégation à partir du volume d'échantillon testé, ce qui ne se produit pas en absence d'agent I. Ces molécules induisent donc une précipitation de la protéine recombinante Tau après une incubation de 60 minutes à 37°C et une centrifugation simple ; la protéine est alors disponible pour une détection par une technique Elisa utilisable en routine dans un laboratoire clinique.

### Exemple 9 : Détection de la protéine alpha-Synucléine par un procédé utilisant un agent précipitant I

### 9.1 Technique d'analyse : Gel de protéine et Western Blot

Les échantillons à analyser sont dénaturés en tampon SDS (125 mM Tris HCl pH 6,8, 20% glycérol, 4 % SDS, 0,02% bleu de bromophénol) (50/50 v/v) à 100°C pendant 5 minutes. Ceux-ci sont ensuite déposés sur un gel d'électrophorèse unidimensionnel de polyacrylamide 12% en présence de sodium dodecyl sulfate (SDS-PAGE). Après migration, deux options sont utilisées :
* le gel de protéine est coloré en Bleu de Coomassie par ajout du gelcode blue stain reagent (Pierce) durant une nuit à température ambiante sous agitation. Après lavages en eau distillée, le gel est incubé en solution de séchage (Pierce) 15 minutes à température ambiante sous agitation puis mis à sécher entre deux feuilles de cellophane au moins une nuit à température ambiante.
* L'analyse est réalisée selon la technique de Western Blotting (Laemmli UK, 1970, Nature, 227: 680-685). Les protéines sont transférées sur membrane de nitrocellulose et immunoblottées à température ambiante pendant une heure avec un anticorps spécifique de la protéine Syn 211 (Zymed). L'anticorps secondaire de détection est un anticorps de chèvre (Jackson) reconnaissant les chaînes lourdes et légères des immunoglobulines G de souris conjugué à la peroxydase de Raifort. La membrane est lavée entre chaque étape en tampon phosphate salin (PBS) avec, puis sans Tween 20 (0,05% p/v). Les signaux sont détectés par chimioluminescence avec le kit super signal (Pierce) et visualisés sur un film radiographique (Pierce).

### 9.2 Détection en présence de l'agent I

Les essais sont réalisés avec 1 µg de protéine Alpha Synucléine (SYN) recombinante (rPeptide) surchargée dans un pool de plasmas humains et avec, comme agent I, soit de la streptomycine, sous forme de sulfate, soit de la triéthylènetétramine ou TET, à différentes concentrations.
Une fois que la protéine recombinante et l'agent I sont mis en contact, le mélange est incubé pendant 60 minutes à 37°C.
Les échantillons sont ensuite centrifugés pendant 10 minutes à 13000 rpm. Les culots sont récupérés et sont séparés des surnageants. Toutes les fractions sont conservées. Les culots sont repris avec 250 µl de tampon SDS. Après dénaturation, toutes les fractions sont analysées par la technique du Western Blotting.

La figure 15 donne les résultats obtenus (représentation schématique, panel haut, et photo, panel bas) sur un gel de protéine après coloration (figure 15A) et après Western Blotting (figure 15B) , obtenu après migration de la protéine SYN dans le plasma après traitement par la TET (pistes 2-5 : culots, 8-11 : sumageants), les pistes 1 et 7 correspondent aux culots et surnageants de l'échantillon témoin sans ajout d'agent I, la piste 6 contient des marqueurs de poids moléculaire et la piste 12 correspondant à la protéine recombinante seule.
La figure 16 donne les résultats obtenus (représentation schématique, panel haut, et photo, panel bas) sur un gel de protéine après coloration (figure 16A) et après Western Blotting (figure 16B) , obtenu après migration de la protéine SYN dans le plasma après traitement par la streptomycine (pistes 2-5 : culots, 8-11 : surnageants), les pistes 1 et 7 correspondent aux culots et surnageants de l'échantillon témoin sans ajout d'agent I, la piste 6 contient des marqueurs de poids moléculaire et la piste 12 correspondant à la protéine recombinante seule.
Les concentrations en agent 1 par rapport aux pistes d'électrophorèse sont telles qu'indiquées dans les tableaux 7 et 8 ci-dessous.

**Tableau 7 (figure 15)**

| **piste** | **Echantillon déposé** | **fraction** |
|---|---|---|
| 1 | Syn recombinante dans plasma | Culot |
| 2 | Syn recombinante dans plasma traitée 50mg/ml TET | Culot |
| 3 | Syn recombinante dans plasma traitée 100mg/ml TET | Culot |
| 4 | Syn recombinante dans plasma traitée 300mg/ml TET | Culot |
| 5 | Syn recombinante dans plasma traitée 500mg/ml TET | Culot |
| 6 | Marqueurs poids moléculaire | NA |
| 7 | Syn recombinante dans plasma | Surnageant |
| 8 | Syn recombinante dans plasma traitée 50 mg/ml TET | Surnageant |
| 9 | Syn recombinante dans plasma traitée 100 mg/ml TET | Surnageant |
| 10 | Syn recombinante dans plasma traitée 300 mg/ml TET | Surnageant |
| 11 | Syn recombinante dans plasma traitée 500 mg/ml TET | Surnageant |
| 12 | Syn recombinante non traitée | Pas de fractionnement |

**Tableau 8 (figure 16)**

| **piste** | **Echantillon déposé** | **fraction** |
|---|---|---|
| 1 | Syn recombinante dans plasma | Culot |
| 2 | Syn recombinante dans plasma traitée 50 mg/ml streptomycine | Culot |
| 3 | Syn recombinante dans plasma traitée 100 mg/ml streptomycine | Culot |
| 4 | Syn recombinante dans plasma traitée 300 mg/ml streptomycine | Culot |
| 5 | Syn recombinante dans plasma traitée 500 mg/ml streptomycine | Culot |
| 6 | Marqueurs poids moléculaire | NA |
| 7 | Syn recombinante dans plasma | Surnageant |
| 8 | Syn recombinante dans plasma traitée 50 mg/ml streptomycine | Surnageant |
| 9 | Syn recombinante dans plasma traitée 100 mg/ml streptomycine | Surnageant |
| 10 | Syn recombinante dans plasma traitée 300 mg/ml streptomycine | Surnageant |
| 11 | Syn recombinante dans plasma traitée 500 mg/ml streptomycine | Surnageant |
| 12 | Syn recombinante non traitée | Pas de fractionnement |

Les résultats montrent que la protéine Syn est retrouvée dans le culot après traitement avec l'agent I, que ce soit la streptomycine ou la TET. Sa présence dans le culot de sédimentation atteste de sa concentration par agrégation à partir du volume d'échantillon testé. Ces molécules induisent donc une précipitation de la protéine recombinante Syn après une incubation de 60 minutes à 37°C et une centrifugation simple. Toutefois cette précipitation n'est pas totale puisqu'on retrouve de la protéine dans la fraction surnageante. Néanmoins cette concentration est suffisante pour permettre sa détection dans un test Elisa utilisable en routine dans un laboratoire clinique.

On remarquera également que cette précipitation est relativement spécifique puisque les protéines plasmatiques sont majoritairement retrouvées dans le surnageant (figure 15A et 16A, pistes 7-12)

### Exemple 10 : Détection de la protéine Alpha-Synueléine par un procédé utilisant un agent précipitant II (procédé non compris dans l'invention)

### 10.1 Technique d'analyse : Gel de protéine et Western Blot

La technique est la même que celle décrite dans l'exemple 9

### 10.2 Technique d'analyse: ELISA

L'analyse est réalisée selon la technique immunoenzymatique classique de type « ELISA sandwich » : human alpha-synuclein ELISAkit (BioSource KHB0061) en suivant les prescriptions du fournisseur.

### 10.3 Détection en présence de l'agent II

Les essais sont réalisés avec 1 µg de protéine Alpha Synucléine (SYN) recombinante (rPeptide) surchargée dans un pool de plasmas humains et avec, comme agent II le calix-arène *p*-sulphonato-3,7-(2-amino-éthyloxy)-calix-[6]-arène (dénommé C6S). Les essais sont réalisés comme indiqué dans l'exemple précédent, à ceci près qu'on a utilisé du C6S préparé comme indiqué dans la demande de brevet WO2004/059322, avec une gamme de concentrations de 0 à 200 mg/ml.

La figure 17 donne les résultats obtenus (représentation schématique, panel haut, et photo, panel bas) sur un gel de protéine, obtenu après migration de la protéine SYN dans le plasma après traitement par les calixarènes. La figure 17A correspond à la fraction culots alors que la figure 17B correspond aux surnageants. Les pistes 1 à 7 correspondent à la protéine Syn traitée par différentes concentrations de calixarènes, la piste 8 contient des marqueurs de poids moléculaire et la piste 9 correspondant à la protéine recombinante non traitée.
Les concentrations en agent II par rapport aux pistes d'électrophorèse sont telles qu'indiquées dans les tableaux 9 et 10 ci-dessous.

**Tableau 9 (figure 17A)**

| **piste** | **Echantillon déposé** | **fraction** |
|---|---|---|
| 1 | Syn recombinante dans plasma | Culot |
| 2 | Syn recombinante dans plasma traitée 2 mg/ml Calixarène | Culot |
| 3 | Syn recombinante dans plasma traitée 5 mg/ml Calixarène | Culot |
| 4 | Syn recombinante dans plasma traitée 10 mg/ml Calixarène | Culot |
| 5 | Syn recombinante dans plasma traitée 50 mg/ml Calixarène | Culot |
| 6 | Syn recombinante dans plasma traitée 100 mg/ml Calixarène | Culot |
| 7 | Syn recombinante dans plasma traitée 200 mg/ml Calixarène | Culot |
| 8 | Marqueurs poids moléculaire | NA |
| 9 | Syn recombinante non traitée | Pas de fractionnement |

**Tableau 10 (figure 17B)**

| **piste** | **Echantillon déposé** | **fraction** |
|---|---|---|
| 1 | Syn recombinante dans plasma | Surnageant |
| 2 | Syn recombinante dans plasma traitée 2 mg/ml Calixarène | Surnageant |
| 3 | Syn recombinante dans plasma traitée 5 mg/ml Calixarène | Surnageant |
| 4 | Syn recombinante dans plasma traitée 10 mg/ml Calixarène | Surnageant |
| 5 | Syn recombinante dans plasma traitée 50 mg/ml Calixarène | Surnageant |
| 6 | Syn recombinante dans plasma traitée 100 mg/ml Calixarène | Surnageant |
| 7 | Syn recombinante dans plasma traitée 200 mg/ml Calixarène | Surnageant |
| 8 | Marqueurs poids moléculaire | NA |
| 9 | Syn recombinante non traitée | Pas de fractionnement |

Les résultats montrent qu'à partir d'une concentration de 10 mg/ml les protéines du plasma sont exclusivement retrouvées dans les culots (dans la limite de sensibilité du gel de protéines).

La figure 18 donne les résultats obtenus (représentation schématique, panel haut, et photo, panel bas) à partir de l'autoradiographie d'un gel d'électrophorèse suivi d'un Western Blotting, obtenu après migration de la protéine SYN dans le plasma après traitement par les calixarènes. La figure 18A correspond à la fraction culots alors que la figure 18B correspond aux surnageants. Les pistes 1 à 7 correspondent à la protéine Syn traitée par différentes concentrations de calixarènes, la piste 8 contient des marqueurs de poids moléculaire et la piste 9 correspondant à la protéine recombinante non traitée. Les concentrations en agent II par rapport aux pistes d'électrophorèse sont telles qu'indiquées dans les tableaux 11 et 12 ci-dessous.

**Tableau 11 (figure 18A)**

| **piste** | **Echantillon déposé** | **fraction** |
|---|---|---|
| 1 | Syn recombinante dans plasma | Culot |
| 2 | Syn recombinante dans plasma traitée 2 mg/ml Calixarène | Culot |
| 3 | Syn recombinante dans plasma traitée 5 mg/ml Calixarène | Culot |
| 4 | Syn recombinante dans plasma traitée 10 mg/ml Calixarène | Culot |
| 5 | Syn recombinante dans plasma traitée 50 mg/ml Calixarène | Culot |
| 6 | Syn recombinante dans plasma traitée 100 mg/ml Calixarène | Culot |
| 7 | Syn recombinante dans plasma traitée 200 mg/ml Calixarène | Culot |
| 8 | Marqueurs poids moléculaire | NA |
| 9 | Syn recombinante non traitée | Pas de fractionnement |

**Tableau 12 (figure 18B)**

| **piste** | **Echantillon déposé** | **fraction** |
|---|---|---|
| 1 | Syn recombinante dans plasma | Surnageant |
| 2 | Syn recombinante dans plasma traitée 2 mg/ml Calixarène | Surnageant |
| 3 | Syn recombinante dans plasma traitée 5 mg/ml Calixarène | Surnageant |
| 4 | Syn recombinante dans plasma traitée 10 mg/ml Calixarène | Surnageant |
| 5 | Syn recombinante dans plasma traitée 50 mg/ml Calixarène | Surnageait |
| 6 | Syn recombinante dans plasma traitée 100 mg/ml Calixarène | Surnageant |
| 7 | Syn recombinante dans plasma traitée 200 mg/ml Calixarène | Surnageant |
| 8 | Marqueurs poids moléculaire | NA |
| 9 | Syn recombinante non traitée | Pas de fractionnement |

Les résultats montrent que la protéine Syn est retrouvée dans le culot après traitement avec l'agent II. Sa présence dans le culot de sédimentation atteste de sa capture à partir du volume d'échantillon testé. Ces molécules induisent donc une capture de la protéine recombinante Syn après une incubation de 60 minutes à 37°C mais ici, de façon inattendue pour un agent de type II, cette capture rend la protéine précipitable par une centrifugation simple. Toutefois cette capture n'est pas totale puisqu'on retrouve de la protéine dans la fraction surnageante. Le maximum de protéine retrouvée dans le culot l'est pour une concentration de calixarène comprise entre 10 et 50 mg/ml
Néanmoins cette concentration est suffisante pour permettre sa détection dans un test Elisa utilisable en routine dans un laboratoire clinique.

### 10.4 Sensibilité de détection en présence de l'agent II

Les essais sont réalisés avec une gamme de protéine Alpha Synucléine (SYN) recombinante (rPeptide) surchargée dans un pool de plasmas humains et avec, comme agent II, le calix-arène *p*-sulphonato-3,7-(2-amino-éthyloxy)-calix-[6]-arène (dénommé C6S). Les essais sont réalisés comme indiqué dans l'exemple précédent, à ceci près qu'on a utilisé du C6S préparé comme indiqué dans la demande de brevet WO2004/059322, avec une concentration de 50 mg/ml.

La figure 19 donne les résultats obtenus (représentation schématique, panel haut, et photo, panel bas) à partir de l'autoradiographie d'un gel d'électrophorèse suivi d'un Western Blotting, obtenu après migration de la protéine SYN dans le plasma après traitement par les calixarènes. La figure 19A correspond à la fraction culots alors que la figure 19B correspond aux surnageants. Les pistes 1 à 8 correspondent à différentes concentrations de la protéine Syn traitée par les calixarènes, les pistes 10 à 12 correspondent à la protéine Syn non traitée et la piste 9 contient des marqueurs de poids moléculaire.
Les concentrations en protéine par rapport aux pistes d'électrophorèse sont telles qu'indiquées dans les tableaux 13 et 14 ci-dessous.

**Tableau 13 (figure 19A)**

| **piste** | **Echantillon déposé** | **fraction** |
|---|---|---|
| 1 | Syn recombinante dans plasma traitée calixarènes: 0 ng/ml | Culot |
| 2 | Syn recombinante dans plasma traitée calixarènes: 10 ng/ml | Culot |
| 3 | Syn recombinante dans plasma traitée calixarènes: 25 ng/ml | Culot |
| 4 | Syn recombinante dans plasma traitée calixarènes: 50 ng/ml | Culot |
| 5 | Syn recombinante dans plasma traitée calixarènes: 100 ng/ml | Culot |
| 6 | Syn recombinante dans plasma traitée calixarènes: 250 ng/ml | Culot |
| 7 | Syn recombinante dans plasma traitée calixarènes: 500 ng/ml | Culot |
| 8 | Syn recombinante dans plasma traitée calixarènes: 1000 ng/ml | Culot |
| 9 | Marqueurs poids moléculaire | NA |
| 10 | Syn recombinante dans plasma non traitée : 0 ng/ml | Pas de fractionnement |
| 11 | Syn recombinante dans plasma non traitée : 10 ng/ml | pas de fractionnement |
| 12 | Syn recombinante dans plasma non traitée : 25 ng/ml | Pas de fractionnement |

**Tableau 14 (figure 19B)**

| **piste** | **Echantillon déposé** | **fraction** |
|---|---|---|
| 1 | Syn recombinante dans plasma traitée calixarènes: 0 ng/ml | Surnageant |
| 2 | Syn recombinante dans plasma traitée calixarènes: 10 ng/ml | Surnageant |
| 3 | Syn recombinante dans plasma traitée calixarènes: 25 ng/ml | Surnageant |
| 4 | Syn recombinante dans plasma traitée calixarènes: 50 ng/ml | Surnageant |
| 5 | Syn recombinante dans plasma traitée calixarènes: 100 ng/ml | Surnageant |
| 6 | Syn recombinante dans plasma traitée calixarènes: 250 ng/ml | Surnageant |
| 7 | Syn recombinante dans plasma traitée calixarènes: 500 ng/ml | Surnageant |
| 8 | Syn recombinante dans plasma traitée calixarènes: 1000 ng/ml | Surnageant |
| 9 | Marqueurs poids moléculaire | NA |
| 10 | Syn recombinante dans plasma non traitée : 50 ng/ml | Pas de fractionnement |
| 11 | Syn recombinante dans plasma non traitée : 100 ng/ml | Pas de fractionnement |
| 12 | Syn recombinante dans plasma non traitée : 250 ng/ml | Pas de fractionnement |

Les résultats montrent que la protéine Syn est retrouvée dans le culot après traitement avec l'agent II, avec une sensibilité de 25 ng/ml. Sa présence dans le culot de sédimentation atteste de sa capture, qui ici a créé les conditions favorables à sa précipitation à partir du volume d'échantillon testé. Ces molécules induisent donc une capture de la protéine recombinante Syn après une incubation de 60 minutes à 37°C et favorisent sa concentration par une centrifugation simple. Cette capture est totale puisqu'on ne retrouve pas la protéine dans la fraction surnageante jusqu'à une concentration de 100 ng/ml.
Ces résultats sont confirmés en test Elisa (voir exemple 10) (figure 20) où une concentration de 25 ng/ml est détectée de manière significative dans le culot par rapport au surnageant

### Exemple 11 : Détection de la protéine Tau totale en présence d'un agent II (procédé non compris dans l'invention)

### Technique d'analyse :

L'analyse est réalisée selon la technique immunoenzymatique classique de type « ELISA sandwich » comme décrite par Kohnken and coll (Neuroscience Letters 287 (2000) 187-190) où les calixarènes remplacent l'anticorps habituellement utilisé en capture.
Le test Elisa repose sur l'utilisation de microplaques activées NHS sur lesquelles sont greffées des calixarènes à une concentration de 0,6 mg/ml durant 2h à température ambiante. Après avoir effectué 3 lavages en eau distillée les plaques sont séchées sous vide 15 minutes à 37°C. Les plaques sont ensuite passivées avec 200 µl/puits de PBS contenant 0,5% lait pendant 1h à 37°C. Après 3 lavages en PBS contenant 0,05% de Tween20, les plaques sont prêtes à l'emploi.
Des extraits de cerveaux de patients atteints (AD+) ou non de la maladie d'Alzheimer (AD-) sont dilués dans un tampon contenant 200 µg/ml d'albumine humaine, 22 µg/ml d'IgG humaine et 0,15 µg/ml d'IgM humaines dans un tampon bicarbonate Krebs-Ringer. Des concentrations de 1 à 10000 ng sont testées ; 100 µl de ces dilutions sont incubées pendant 1h30 à 37°C. 100 µl d'un anticorps monoclonal de souris spécifique de la protéine Tau T14 (Zymed) à la concentration de 3 µg/ml diluée en tampon PBS contenant 0,05% de Tween20 sont ensuite incubés durant 1h à 37°C.
Afin de reconnaître l'anticorps anti T14 fixé, un anticorps de chèvre anti IgG de souris marqué à la peroxydase (Jackson) est utilisé à 0,5 µg/ml en tampon PBS contenant 0,05% de Tween20 durant 45 minutes à 37°C. Un réactif Lumiglo (Kirkegaard and Perry laboratories, US) est ajouté dans chaque puits et la chemiluminescence lue dans un luminomètre (Berthold, Centro LB 960). Les données obtenues sont sous forme d'unité relative de lumière (RLU). Entre chaque étape décrite précédemment les puits sont lavés avec du tampon PBS contenant 0,05% de Tween20.

Les résultats de la figure 21 montrent que la protéine Tau est parfaitement capturée par les calixarènes jusqu'à une concentration de 1 ng/ml. Cette capture permet la détection par un anticorps spécifique dans un format de test Elisa. L'homme de l'art aura remarqué un « effet crochet » pour les fortes concentrations de protéine, phénomène facilement solutionné lors d'une optimisation effectuée par un expert. Le différentiel obtenu entre les patients atteints de la maladie d'Alzheimer (AD+) et les patients non atteints (AD-) permet l'utilisation de ce test en routine dans un laboratoire.

### Exemple 12 : Détection de la protéine syn en présence des agents I et II

### Technique d'analyse

La première analyse est réalisée selon la technique immunoenzymatique classique de type « ELISA sandwich » : human alpha-synuclein ELISA kit (BioSource KHB0061) en suivant les prescriptions du fournisseur.

La seconde analyse est réalisée selon la technique immunoenzymatique classique de type « ELISA sandwich » comme décrite par Kohnken and coll (Neuroscience Letters 287 (2000) 187-190) où les calixarènes remplacent l'anticorps habituellement utilisé en capture.
Le test Elisa repose sur l'utilisation de microplaques activées NHS sur lesquelles sont greffées des calixarènes (agent II) à une concentration de 0,6 mg/ml durant deux heures à température ambiante. Après avoir effectué 3 lavages en eau distillée les plaques sont séchées sous vide 15 minutes à 37°C. Les plaques sont ensuite passivées avec 200 µl/puits de PBS contenant 0,5% lait pendant 1h à 37°C. Après 3 lavages en PBS contenant 0,05% de Tween20, les plaques sont prêtes à l'emploi.
Les essais sont réalisés avec des extraits de cerveaux de patients atteints de la maladie d'Alzheimer (20 µg/ml) et une protéine Alpha Synucléine (SYN) recombinante (rPeptide) (25 ng/ml) sont surchargés dans un pool de plasmas humains.
Les échantillons sont précipités en présence d'agent I (streptomycine à 500 mg/ml). Une fois que l'échantillon et l'agent I sont mis en contact, le mélange est incubé pendant 60 minutes à 37°C.
Les échantillons sont ensuite centrifugés pendant 10 minutes à 13000 rpm. Les culots sont récupérés et les culots sont séparés des surnageants. Toutes les fractions sont conservées. Les culots sont repris avec 25 µl de guanidine-HCl 6M, chauffés ou non durant 1 minute à 90°C puis dilués dans 225 µl de tampon tris-maléate. Les échantillons sont alors prêts pour être déposés sur plaques.
La figure 22 montre les résultats obtenus après précipitation avec l'agent I et détection avec une technique Elisa classique avec capture anticorps (première analyse; sans utilisation d'agent II). Les résultats montrent que la protéine recombinante est détectée dans le plasma après précipitation par l'agent I; toutefois sa précipitation sous forme agrégée ne permet pas une détection optimale. La précipitation n'est également pas totale puisque la protéine est détectée dans le surnageant et que cette forme monomérique est parfaitement capturée par les anticorps. Par contre dans l'extrait de cerveaux, où la protéine est majoritairement sous forme agrégée on voit tout de suite que la détection dans l'échantillon non traitée est faible et qu'après traitement par un agent I, la protéine n'est détectée ni dans le culot ni dans le surnageant dans les conditions opératoires de cet exemple.
La figure 23 montre les résultats obtenus après précipitation sous forme agrégée avec l'agent 1 et détection avec une technique ELISA classique avec capture par l'agent II. Les résultats montrent que la protéine recombinante et l'extrait de cerveau sont peu ou pas détectés s'ils ne sont pas traités. Par contre après action de l'agent I, on peut très facilement voir l'intérêt d'utiliser l'agent II comme outil de capture. En effet tant dans le cas de la protéine recombinante que de l'extrait de cerveau, la protéine Syn sous sa forme agrégée est parfaitement détectée dans le culot de précipitation. De plus l'étape de chauffage permet d'améliorer le signal obtenu.
On peut donc conclure que l'association agent I/agent II permet d'améliorer la détection de la protéine alpha-synucléine dans un échantillon tel que le plasma.

### Exemple 13 : Détection de la protéine Tau Phosphorylée en présence des agents I et II

La technique d'analyse est la même que celle décrite dans l'exemple 8, sauf que les anticorps de capture sont remplacés par des calixarènes (agent II) comme décrit dans l'exemple 12. L'anticorps de détection utilisé est un anticorps monoclonal développé par bioMérieux et utilisé conjugué à la peroxydase à une concentration de 1 µg/ml. Cet anticorps monoclonal a été obtenu après immunisation par un peptide phosphorylé, dérivé de la protéine Tau phosphorylée en position 231.
L'échantillon utilisé est un extrait de cerveau de patient ayant la maladie d'Alzheimer (AD pos) et surchargé dans un pool de plasma de patients sains. Les essais sont réalisés avec 25 ng d'extrait de cerveaux et avec, comme agent I, de la streptomycine, sous forme de sulfate, à différentes concentrations.
Une fois que l'échantillon et l'agent I sont mis en contact, le mélange est incubé pendant 60 minutes à 37°C. Les échantillons sont ensuite centrifugés pendant 10 minutes à 13000 rpm. Les culots sont récupérés et les culots sont séparés des surnageants. Toutes les fractions sont conservées. Les culots sont repris avec 250 µl de tampon tris-maléate (sauf indication contraire). Les échantillons sont alors prêts pour être déposés sur plaques.
Les résultats de la figure 24 montrent que la protéine Tau phosphorylée est difficilement détectée dans un échantillon de plasma non traité. Par contre il apparaît clairement qu'après agrégation par l'agent I, sa détection dans un test Elisa, où la capture est réalisée *via* un agent II, est rendue possible dans le culot. On peut donc conclure que l'association agent I/agent II permet d'améliorer la détection de la protéine Tau phosphorylée dans un échantillon tel que le plasma.

## Revendications

1. Procédé de détection d'au moins une forme protéique circulante d'agrégation (FPCA) liée aux maladies neurodégénératives non infectieuses dans un échantillon biologique d'origine humaine susceptible de contenir desdites FPCA, lesdites FCPA contenant :
a) des acides aminés dont les chaînes latérales ont soit une fonction acide, soit une fonction accepteur de liaison hydrogène, et qui sont présents globalement au moins 4 fois dans une séquence peptidique de longueur inférieure ou égale à 20 acides aminés et
b) un minimum de trois acides aminés basiques dans une séquence peptidique de longueur inférieure ou égale à quinze acides aminés,
**caractérisé en ce qu'**il met en oeuvre un agent 1 non protéique produisant l'agrégation des formes circulantes des protéines non infectieuses impliquées dans des processus d'agrégation pathologique du système nerveux central, qui est choisi parmi la rifampicine, l'isoniazide, l'éthambutol, la triéthylènetétramine, la bis-3-aminopropylamine, le tétrachlorhydrate de spermine, le sesquisulfate dé dihydrostreptomycine et la streptomycine, et un agent II non protéique de capture des agrégats naturels de FPCA ou induits par lesdits agents I, qui est un métacyclophane ou un glycosaminoglycane, et **en ce qu'**il met en oeuvre la révélation de la présence desdits FCPA.

2. Procédé de détection d'au moins une FPCA selon la revendication 1, **caractérisé en ce que** ledit agent I est ajouté audit échantillon biologique pour l'agrégation des FPCA avant ledit agent II.

3. Procédé de détection d'au moins une FPCA selon la revendication 2, **caractérisé en ce qu'**il comprend les étapes consistant à :
a) ajouter audit échantillon ledit agent I pour agréger les FPCA,
b) mettre le mélange ainsi obtenu en présence dudit agent II pour capturer lesdits agrégats de FPCA et
c) révéler la présence des FPCA.

4. Procédé de détection d'au moins une FPCA selon l'une quelconque des revendications 1 à 3, **caractérisé en ce qu'**il comprend au moins l'une des étapes supplémentaires i) et ii) suivantes consistant à :
i) séparer les agrégats de FPCA du mélange réactionnel et
ii) dénaturer les agrégats de FPCA,
ces étapes étant incluses le cas échéant entre l'étape a) et l'étape b).

5. Procédé de détection d'au moins une FPCA selon l'une quelconque des revendications 1 à 4, **caractérisé en ce qu'**on ajoute au moins un partenaire de liaison spécifique des FPCA pour une réaction immunologique entre partenaire de liaison spécifique des FPCA et les FPCA, le cas échéant dans l'étape c).

6. Procédé de détection d'au moins une FPCA selon l'une quelconque des revendications 1 à 5, **caractérisé en ce que** ledit agent II est lié à un support solide.

7. Procédé de détection d'au moins une FPCA selon l'une quelconque des revendications 1 à 6, **caractérisé en ce que** le métacyclophane est le *p*-sulphonato-3,7-(2-aminoéthyloxy)-calix-[6]-arène.

8. Procédé de détection d'au moins une FPCA liées aux maladies neurodégénératives non infectieuses, lesdites FCPA contenant des acides aminés dont les chaînes latérales ont soit une fonction acide, soit une fonction accepteur de liaison hydrogène, et qui sont présents globalement au moins 4 fois dans une séquence peptidique de longueur inférieure ou égale à 20 acides aminés, **caractérisé en ce qu'**il comprend l'étape de mise en présence d'un échantillon biologique, issu ou obtenu d'un organisme humain, avec un agent I non protéique produisant l'agrégation des formes circulantes des protéines non infectieuses impliquées dans des processus d'agrégation pathologique du système nerveux central, qui est choisi parmi la rifampicine, l'isoniazide, l'éthambutol, la triéthylènetétramine, la bis-3-aminopropylamine, le tétrachlorhydrate de spermine, le sesquisulfate de dihydrostreptomycine et la streptomycine, et **en ce qu'**il met en oeuvre la révélation de la présence desdits FCPA.

9. Procédé de détection d'au moins une FPCA selon la revendication 8, **caractérisé en ce qu'**on ajoute au moins un partenaire de liaison spécifique des FPCA pour une réaction immunologique entre partenaire de liaison spécifique des FPCA et les FPCA.

10. Utilisation d'un kit de diagnostic comprenant un agent I non protéique produisant l'agrégation des formes circulantes des protéines non infectieuses impliquées dans des processus d'agrégation pathologique du système nerveux central, qui est choisi parmi la rifampicine, l'isoniazide, l'éthambutol, la triéthylènetétramine, la bis-3-aminopropylamine, le tétrachlorhydrate de spermine, le sesquisulfate de dihydrostreptomycine et la streptomycine pour la détection d'au moins une FPCA liées aux maladies neurodégénératives non infectieuses.

11. Utilisation d'un kit selon la revendication 10, **caractérisé en ce qu'**il comprend également un métacyclophane ou un glycosaminoglycane.

## Claims

1. A method for detecting at least one aggregate-forming circulating protein form associated with noninfectious neurodegenerative diseases (FCPA) in a biological sample of human origin that may contain said aggregate-forming circulating protein forms, said FCPA containing:
a) amino acids whose side chains have either an acid function, or a hydrogen-bond-acceptor function, being present overall at least 4 times in a peptide sequence of length less than or equal to 20 amino acids and
b) a minimum of three basic amino acids in a peptide sequence of length less than or equal to fifteen amino acids,
**characterized in that** it uses a non-protein agent I producing aggregation of the circulating forms of the noninfectious proteins involved in pathological aggregation processes of the central nervous system, which is chosen from rifampicine, isoniazide, éthambutol, triethylenetetramine, bis-3-aminopropylamine, spermine tetrahydrochlorate, dihydrostreptomycine sesquisulfate and streptomycine, and a non-protein agent II for capturing the natural aggregates of FCPA or the aggregates induced by said agents I, which is a metacyclophane or a glycosaminoglycane, and **in that** it carries out revealing the presence of said FCPA.

2. The method for detecting at least one FCPA as claimed in claim 1, **characterized in that** said agent I is added to said biological sample in order to aggregate the FCPA, before said agent II.

3. The method for detecting at least one FCPA as claimed in claim 2, **characterized in that** it comprises the steps consisting in:
a) adding said agent I to said sample in order to aggregate the FCPA,
b) bringing the mixture thus obtained together with said agent II in order to capture said aggregates of FCPA, and
c) revealing the presence of the FCPA.

4. The method for detecting at least one FCPA as claimed in any one of claims 1 to 3, **characterized in that** it comprises at least one of the following additional steps i) and ii) consisting in:
i) separating the aggregates of FCPA from the reaction mixture, and
ii) denaturing the aggregates of FCPA,
these steps being included, where appropriate, between step a) and step b).

5. The method for detecting at least FCPA as claimed in any one of claims 1 to 4, **characterized in that** at least one binding partner specific for the FCPA is added for an immunoreaction between the binding partner specific for the FCPA and the FCPA where appropriate in step c).

6. The method for detecting at least one FCPA as claimed in any one of claims 1 to 5, **characterized in that** said agent II is bound to a solid support.

7. The method for detecting at least one FCPA as claimed in any one of claims 1 to 6, **characterized in that** the metacyclophane is p-sulphonato-3,7-(2-aminoethyloxy)-calix-[6]-arene.

8. A method for detecting at least one FCPA associated with noninfectious neurodegenerative diseases, said FCPA containing amino acids whose side chains have either an acid function, or a hydrogen-bond-acceptor function, and being present overall at least 4 times in a peptide sequence of length less than or equal to 20 amino acids, **characterized in that** it comprises the step of bringing a biological sample, derived or obtained from a human organism, together with a non-protein agent I producing aggregation of the circulating forms of the noninfectious proteins involved in pathological aggregation processes of the central nervous system, which is chosen from rifampicine, isoniazide, éthambutol, triethylenetetramine, bis-3-aminopropylamine, spermine tetrahydrochlorate, dihydrostreptomycine sesquisulfate and streptomycine, and **in that** it carries out revealing the presence of said FCPA.

9. The method for detecting at least one FCPA as claimed in claim 8, **characterized in that** at least one binding partner specific for the FCPA is added for an immunoreaction between the binding partner specific for the FCPA and the FCPA.

10. The use of a diagnostic kit comprising a non-protein agent I producing aggregation of the circulating forms of the noninfectious proteins involved in pathological aggregation processes of the central nervous system, which is chosen from rifampicine, isoniazide, éthambutol, triethylenetetramine, bis-3-aminopropylamine, spermine tetrahydrochlorate, dihydrostreptomycine sesquisulfate and streptomycine, for detecting at least one FCPA associated with noninfectious neurodegenerative diseases.

11. The use of a kit according to claim 10, **characterized in that** it also comprises a metacyclophane or a glycosaminoglycane.

## Patentansprüche

1. Verfahren zum Nachweis mindestens einer zirkulierenden aggregatbildenden Proteinform (FPCA), die mit nicht-infektiösen neurodegenerativen Erkrankungen in Zusammenhang steht, in einer biologischen Probe humanen Ursprungs, die diese FPCA enthalten kann, wobei die FPCA Folgendes enthalten:
a) Aminosäuren, deren Seitenketten entweder eine Säurefunktion oder eine Akzeptorfunktion für eine Wasserstoffbindung besitzen und die im Großen und Ganzen mindestens 4 Mal in einer Peptidsequenz mit einer Länge von weniger oder gleich 20 Aminosäuren vorliegen, und
b) mindestens drei basische Aminosäuren in einer Peptidsequenz mit einer Länge von weniger oder gleich fünfzehn Aminosäuren,
**dadurch gekennzeichnet, dass** es ein nicht-proteinartiges Mittel I verwendet, das die Aggregation der zirkulierenden Formen nicht-infektiöser Proteine hervorruft, die an pathologischen Aggregationsvorgängen des Zentralnervensystems beteiligt sind, und das ausgewählt ist aus Rifampicin, Isoniazid, Ethambutol, Triethylentetramin, Bis-3-aminopropylamin, Spermintetrahydrochlorid, Dihydrostreptomycinsesquisulfat und Streptomycin, und ein nicht-proteinartiges Mittel II zum Einfangen der FPCA-Aggregate, die natürlich sind oder durch die Mittel I induziert worden sind, bei dem es sich um ein Metacyclophan oder ein Glycosaminoglykan handelt, und **dadurch**, dass es die Sichtbarmachung des Vorliegens der FCPA einsetzt.

2. Verfahren zum Nachweis mindestens einer FPCA nach Anspruch 1, **dadurch gekennzeichnet, dass** das Mittel I zu der biologischen Probe für die Aggregation der FPCA vor dem Mittel II zugegeben wird.

3. Verfahren zum Nachweis mindestens einer FPCA nach Anspruch 2, **dadurch gekennzeichnet, dass** es folgende Schritte umfasst:
a) Zugeben des Mittels I zu der Probe, um die FPCA zu aggregieren,
b) Zusammenbringen des so erhaltenen Gemischs mit dem Mittel II, um die FPCA-Aggregate einzufangen, und
c) Sichtbarmachen des Vorliegens der FPCA.

4. Verfahren zum Nachweis mindestens einer FPCA nach einem der Ansprüche 1 bis 3, **dadurch gekennzeichnet, dass** es mindestens einen der folgenden zusätzlichen Schritte i) und ii) umfasst:
i) Abtrennen der FPCA-Aggregate aus dem Reaktionsgemisch und
ii) Denaturieren der FPCA-Aggregate,
wobei diese Schritte gegebenenfalls zwischen den Schritt a) und den Schritt b) eingefügt werden.

5. Verfahren zum Nachweis mindestens einer FPCA nach einem der Ansprüche 1 bis 4, **dadurch gekennzeichnet, dass** man, gegebenenfalls im Schritt c), mindestens einen von FPCA spezifischen Bindungspartner für eine Immunreaktion zwischen dem von FPCA spezifischen Bindungspartner und den FPCA zugibt.

6. Verfahren zum Nachweis mindestens einer FPCA nach einem der Ansprüche 1 bis 5, **dadurch gekennzeichnet, dass** das Mittel II an einen festen Träger gebunden ist.

7. Verfahren zum Nachweis mindestens einer FPCA nach einem der Ansprüche 1 bis 6, **dadurch gekennzeichnet, dass** es sich bei dem Metacyclophan um p-Sulfonato-3,7-(2-aminoethyloxy)-calix-[6]-aren handelt.

8. Verfahren zum Nachweis mindestens einer FPCA, die mit nicht-infektiösen neurodegenerativen Erkrankungen in Zusammenhang steht, wobei die FPCA Aminosäuren enthalten, deren Seitenketten entweder eine Säurefunktion oder eine Akzeptorfunktion für eine Wasserstoffbindung besitzen und die im Großen und Ganzen mindestens 4 Mal in einer Peptidsequenz mit einer Länge von weniger oder gleich 20 Aminosäuren vorliegen, **dadurch gekennzeichnet, dass** es den Schritt umfasst, bei dem eine biologische Probe, die aus einem menschlichen Organismus stammt oder von diesem erhalten wird, mit einem nicht-proteinartigen Mittel I zusammengebracht wird, das die Aggregation der zirkulierenden Formen nicht-infektiöser Proteine hervorruft, die an pathologischen Aggregationsvorgängen des Zentralnervensystems beteiligt sind, und das ausgewählt ist aus Rifampicin, Isoniazid, Ethambutol, Triethylentetramin, Bis-3-aminopropylamin, Spermintetrahydrochlorid, Dihydrostreptomycinsesquisulfat und Streptomycin, und **dadurch**, dass es das Sichtbarmachen des Vorliegens der FPCA einsetzt.

9. Verfahren zum Nachweis mindestens einer FPCA nach Anspruch 8, **dadurch gekennzeichnet, dass** man mindestens einen von FPCA spezifischen Bindungspartner für eine Immunreaktion zwischen dem von FPCA spezifischen Bindungspartner und den FPCA zugibt.

10. Verwendung eines Diagnosekits, umfassend ein nicht-proteinartiges Mittel I, das die Aggregation der zirkulierenden Formen nicht-infektiöser Proteine hervorruft, die an pathologischen Aggregationsvorgängen des Zentralnervensystems beteiligt sind, und das ausgewählt ist aus Rifampicin, Isoniazid, Ethambutol, Triethylentetramin, Bis-3-aminopropylamin, Spermintetrahydrochlorid, Dihydrostreptomycinsesquisulfat und Streptomycin, zum Nachweis mindestens einer FPCA, die mit nicht-infektiösen neurodegenerativen Erkrankungen in Zusammenhang steht.

11. Verwendung eines Kits nach Anspruch 10, **dadurch gekennzeichnet, dass** es außerdem ein Metacyclophan oder ein Glycosaminoglykan umfasst.


### SEQUENCE LISTING

<110> bioMérieux
   Centre National de la Recherche Scientifique
   Université Claude Bernard Lyon 1
<120> Procédé de détection des FPCA utilisant un agent d'agrégation des FPCA et un agent de capture des agrégats formés
<130> Neuroninf
<160> 6
<170> PatentIn version 3.3
<210> 1
   <211> 140
   <212> PRT
   <213> human
<400> 1
<210> 2
   <211> 770
   <212> PRT
   <213> human
<400> 2
<210> 3
   <211> 465
   <212> PRT
   <213> human
<400> 3
   Met Ile Val Phe Val Arg Phe Asn Ser Ser His Gly Phe Pro Val Glu
   1               5                   10                  15
   Val Asp Ser Asp Thr Ser Ile Phe Gln Leu Lys Glu Val Val Ala Lys
               20                  25                  30
   Arg Gln Gly Val Pro Ala Asp Gln Leu Arg Val Ile Phe Ala Gly Lys
           35                  40                  45
   Glu Leu Arg Asn Asp Trp Thr Val Gln Asn Cys Asp Leu Asp Gln Gln
       50                  55                  60
   Ser Ile Val His Ile Val Gln Arg Pro Trp Arg Lys Gly Gln Glu Met
   65                  70                  75                  80
   Asn Ala Thr Gly Gly Asp Asp Pro Arg Asn Ala Ala Gly Gly Cys Glu
                   85                  90                  95
   Arg Glu Pro Gln Ser Leu Thr Arg Val Asp Leu Ser Ser Ser Val Leu
               100                 105                 110
   Pro Gly Asp Ser Val Gly Leu Ala Val Ile Leu His Thr Asp Ser Arg
           115                 120                 125
   Lys Asp Ser Pro Pro Ala Gly Ser Pro Ala Gly Arg Ser Ile Tyr Asn
       130                 135                 140
   Ser Phe Tyr Val Tyr Cys Lys Gly Pro Cys Gln Arg Val Gln Pro Gly
   145                 150                 155                 160
   Lys Leu Arg Val Gln Cys Ser Thr Cys Arg Gln Ala Thr Leu Thr Leu
   165 170 175
   Thr Gln Gly Pro Ser Cys Trp Asp Asp Val Leu Ile Pro Asn Arg Met
   180 185 190
   Ser Gly Glu Cys Gln Ser Pro His Cys Pro Gly Thr Ser Ala Glu Phe
   195 200 205
   Phe Phe Lys Cys Gly Ala His Pro Thr Ser Asp Lys Glu Thr Pro Val
   210 215 220
   Ala Leu His Leu Ile Ala Thr Asn Ser Arg Asn Ile Thr Cys Ile Thr
   225 230 235 240
   Cys Thr Asp Val Arg Ser Pro Val Leu Val Phe Gln Cys Asn Ser Arg
   245 250 255
   His Val Ile Cys Leu Asp Cys Phe His Leu Tyr Cys Val Thr Arg Leu
   260 265 270
   Asn Asp Arg Gln Phe Val His Asp Pro Gln Leu Gly Tyr Ser Leu Pro
   275 280 285
   Cys Val Ala Gly Cys Pro Asn Ser Leu Ile Lys Glu Leu His His Phe
   290 295 300
   Arg Ile Leu Gly Glu Glu Gln Tyr Asn Arg Tyr Gln Gln Tyr Gly Ala
   305 310 315 320
   Glu Glu Cys Val Leu Gln Met Gly Gly Val Leu Cys Pro Arg Pro Gly
   325 330 335
   Cys Gly Ala Gly Leu Leu Pro Glu Pro Asp Gln Arg Lys Val Thr Cys
   340 345 350
   Glu Gly Gly Asn Gly Leu Gly Cys Gly Phe Ala Phe Cys Arg Glu Cys
   355 360 365
   Lys Glu Ala Tyr His Glu Gly Glu Cys Ser Ala Val Phe Glu Ala Ser
   370 375 380
   Gly Thr Thr Thr Gln Ala Tyr Arg Val Asp Glu Arg Ala Ala Glu Gln
   385 390 395 400
   Ala Arg Trp Glu Ala Ala Ser Lys Glu Thr Ile Lys Lys Thr Thr Lys
   405 410 415
   Pro Cys Pro Arg Cys His Val Pro Val Glu Lys Asn Gly Gly Cys Met
   420 425 430
   His Met Lys Cys Pro Gln Pro Gln Cys Arg Leu Glu Trp Cys Trp Asn
   435 440 445
   Cys Gly Cys Glu Trp Asn Arg Val Cys Met Gly Asp His Trp Phe Asp
   450 455 460
   Val
   465
<210> 4
   <211> 758
   <212> PRT
   <213> human
<400> 4
   Met Ala Glu Pro Arg Gln Glu Phe Glu Val Met Glu Asp His Ala Gly
   1               5                   10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Glu Ser Pro Leu
           35                  40                  45
   Gln Thr Pro Thr Glu Asp Gly Ser Glu Glu Pro Gly Ser Glu Thr Ser
       50                  55                  60
   Asp Ala Lys Ser Thr Pro Thr Ala Glu Asp Val Thr Ala Pro Leu Val
   65                  70                  75                  80
   Asp Glu Gly Ala Pro Gly Lys Gln Ala Ala Ala Gln Pro His Thr Glu
                   85                  90                  95
   Ile Pro Glu Gly Thr Thr Ala Glu Glu Ala Gly Ile Gly Asp Thr Pro
               100                 105                 110
   Ser Leu Glu Asp Glu Ala Ala Gly His Val Thr Gln Glu Pro Glu Ser
           115                 120                 125
   Gly Lys Val Val Gln Glu Gly Phe Leu Arg Glu Pro Gly Pro Pro Gly
       130                 135                 140
   Leu Ser His Gln Leu Met Ser Gly Met Pro Gly Ala Pro Leu Leu Pro
   145                 150                 155                 160
   Glu Gly Pro Arg Glu Ala Thr Arg Gln Pro Ser Gly Thr Gly Pro Glu
                   165                 170                 175
   Asp Thr Glu Gly Gly Arg His Ala Pro Glu Leu Leu Lys His Gln Leu
               180                 185                 190
   Leu Gly Asp Leu His Gln Glu Gly Pro Pro Leu Lys Gly Ala Gly Gly
           195                 200                 205
   Lys Glu Arg Pro Gly Ser Lys Glu Glu Val Asp Glu Asp Arg Asp Val
       210                 215                 220
   Asp Glu Ser Ser Pro Gln Asp Ser Pro Pro Ser Lys Ala Ser Pro Ala
   225                 230                 235                 240
   Gln Asp Gly Arg Pro Pro Gln Thr Ala Ala Arg Glu Ala Thr Ser Ile
                   245                 250                 255
   Pro Gly Phe Pro Ala Glu Gly Ala Ile Pro Leu Pro Val Asp Phe Leu
               260                 265                 270
   Ser Lys Val Ser Thr Glu Ile Pro Ala Ser Glu Pro Asp Gly Pro Ser
           275                 280                 285
   Val Gly Arg Ala Lys Gly Gln Asp Ala Pro Leu Glu Phe Thr Phe His
   290 295 300
   Val Glu Ile Thr Pro Asn Val Gln Lys Glu Gln Ala His Ser Glu Glu
   305 310 315 320
   His Leu Gly Arg Ala Ala Phe Pro Gly Ala Pro Gly Glu Gly Pro Glu
   325 330 335
   Ala Arg Gly Pro Ser Leu Gly Glu Asp Thr Lys Glu Ala Asp Leu Pro
   340 345 350
   Glu Pro Ser Glu Lys Gln Pro Ala Ala Ala Pro Arg Gly Lys Pro Val
   355 360 365
   Ser Arg Val Pro Gln Leu Lys Ala Arg Met Val Ser Lys Ser Lys Asp
   370 375 380
   Gly Thr Gly Ser Asp Asp Lys Lys Ala Lys Thr Ser Thr Arg Ser Ser
   385 390 395 400
   Ala Lys Thr Leu Lys Asn Arg Pro Cys Leu Ser Pro Lys Leu Pro Thr
   405 410 415
   Pro Gly Ser Ser Asp Pro Leu Ile Gln Pro Ser Ser Pro Ala Val Cys
   420 425 430
   Pro Glu Pro Pro Ser Ser Pro Lys His Val Ser Ser Val Thr Ser Arg
   435 440 445
   Thr Gly Ser Ser Gly Ala Lys Glu Met Lys Leu Lys Gly Ala Asp Gly
   450 455 460
   Lys Thr Lys Ile Ala Thr Pro Arg Gly Ala Ala Pro Pro Gly Gln Lys
   465 470 475 480
   Gly Gln Ala Asn Ala Thr Arg Ile Pro Ala Lys Thr Pro Pro Ala Pro
   485 490 495
   Lys Thr Pro Pro Ser Ser Gly Glu Pro Pro Lys Ser Gly Asp Arg Ser
   500 505 510
   Gly Tyr Ser Ser Pro Gly Ser Pro Gly Thr Pro Gly Ser Arg Ser Arg
   515 520 525
   Thr Pro Ser Leu Pro Thr Pro Pro Thr Arg Glu Pro Lys Lys Val Ala
   530 535 540
   Val Val Arg Thr Pro Pro Lys Ser Pro Ser Ser Ala Lys Ser Arg Leu
   545 550 555 560
   Gln Thr Ala Pro Val Pro Met Pro Asp Leu Lys Asn Val Lys Ser Lys
   565 570 575
   Ile Gly Ser Thr Glu Asn Leu Lys His Gln Pro Gly Gly Gly Lys Val
   580 585 590
   Gln Ile Ile Asn Lys Lys Leu Asp Leu Ser Asn Val Gln Ser Lys Cys
   595 600 605
   Gly Ser Lys Asp Asn Ile Lys His Val Pro Gly Gly Gly Ser Val Gln
   610 615 620
   Ile Val Tyr Lys Pro Val Asp Leu Ser Lys Val Thr Ser Lys Cys Gly
   625 630 635 640
   Ser Leu Gly Asn Ile His His Lys Pro Gly Gly Gly Gln Val Glu Val
   645 650 655
   Lys Ser Glu Lys Leu Asp Phe Lys Asp Arg Val Gln Ser Lys Ile Gly
   660 665 670
   Ser Leu Asp Asn Ile Thr His Val Pro Gly Gly Gly Asn Lys Lys Ile
   675 680 685
   Glu Thr His Lys Leu Thr Phe Arg Glu Asn Ala Lys Ala Lys Thr Asp
   690 695 700
   His Gly Ala Glu Ile Val Tyr Lys Ser Pro Val Val Ser Gly Asp Thr
   705 710 715 720
   Ser Pro Arg His Leu Ser Asn Val Ser Ser Thr Gly Ser Ile Asp Met
   725 730 735
   Val Asp Ser Pro Gln Leu Ala Thr Leu Ala Asp Glu Val Ser Ala Ser
   740 745 750
   Leu Ala Lys Gln Gly Leu
   755
<210> 5
   <211> 40
   <212> PRT
   <213> human
<400> 5
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 6
   <211> 42
   <212> PRT
   <213> human
<400> 6